(19) Europäisches Patentamt
European Patent Office
Office européen des brevets

(11) Publication number: **0 344 343 B1**

(12) **EUROPEAN PATENT SPECIFICATION**

(45) Date of publication of patent specification: **21.04.93**

(51) Int. Cl.5: **C07C 315/00**, C07C 317/00, C09B 62/006, D06P 1/02

(21) Application number: **88108741.5**

(22) Date of filing: **01.06.88**

# (54) Process for the regiospecific sulfonation of 2-amino-naphtalenes substituted in the 5- or 7-position by a fibre reactive vs-group, the novel 5-sulfo-substituted fiber-reactive 2-amino-naphthalene, and fibre-reactive azo dyes thereof.

(43) Date of publication of application:
**06.12.89 Bulletin 89/49**

(45) Publication of the grant of the patent:
**21.04.93 Bulletin 93/16**

(84) Designated Contracting States:
**BE CH DE FR GB LI**

(56) References cited:
**EP-A- 0 144 701**
**DE-A- 1 943 904**
**FR-A- 2 360 539**
**FR-A- 2 436 169**

(73) Proprietor: **HOECHST CELANESE CORPORATION**
**Route 202-206 North**
**Somerville, N.J. 08876(US)**

(72) Inventor: **Phillips, Thomas S.**
**62 West Warwick Avenue**
**West Warwick, RI 02893(US)**
Inventor: **Corso, Antony J.**
**5 Crocus Court**
**Coventry, RI 02816(US)**

(74) Representative: **Kockläuner, Reinhard, Dr. et al**
**HOECHST AKTIENGESELLSCHAFT Zentrale Patentabteilung Postfach 80 03 20**
**W-6230 Frankfurt am Main 80 (DE)**

EP 0 344 343 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention is in the field of intermediates suitable for the synthesis of dyestuffs, and in the field of fiber reactive dyestuffs.

Sulfonated naphthylamines containing the fiber reactive beta-hydroxyethylsulfone group or its reactive equivalent are important starting materials in the preparation of fiber reactive, water soluble azo dyestuffs. The sulfonation of such substituted naphthylamines is well known in the art. The sulfonation is carried by reacting the substituted naphthylamine with fuming sulfuric acid (oleum) in sulfuric acid usually at elevated temperature.

It is well known in the literature that the sulfonation of aminonaphthalenes and aminonaphthalene monosulfonic acids results in a mixture of isomeric products (see e.g. Hans Cerfontain, Mechanistic Aspects of Aromatic Sulfonation and Desulfonation, John Wiley & Sons, N.Y., N.Y. (1968), Chapter 7). Isomeric sulfonation products are also produced when a naphthylamine substituted with the beta-hydroxyethylsulfone group is sulfonated. These isomeric mixtures of the sulfonation reaction are separated in commercial manufacturing processes by the selective crystallization of one isomer over the other after drowning the reaction solution in ice and water usually containing an inorganic salt such as sodium chloride.

Furthermore, German Auslegeschrift No. 1,943,904 mentions that the 2-amino-6-($\beta$-sulfatoethylsulfonyl)-8-sulfonaphthalene and 2-amino-8-($\beta$-sulfatoethylsulfonyl)-6-sulfonaphthalene compounds can be prepared by sulfonation of the corresponding 2-amino-6- or -8-($\beta$-hydroxyethylsulfonyl)-naphthalene, or alternatively of the 2-acetylamino-6- or -8-($\beta$-hydroxyethylsulfonyl)-naphthalene under subsequent deacetylation (see col. 4, lines 3 to 28). No hint can be found whether the use of the starting aminonaphthalene in the acetylated form effects any advantages in respect to the yield of the desired sulfo-substituted final compound; rather it is obvious that the acetylamino derivative has only be used in order to protect the amino group in its precursor against ethoxylation when the sulfinic acid precursor is reacted with ethylene oxide. Consequently, although this prior art discloses a general procedure for the preparation of sulfonated beta-sulfatoethylsulfonyl aminonaphthalenes, there is no appreciation or recognition of the regiospecificity of this known process.

It has now been found, according to the present invention, that 5- and 7- sulfonyl-substituted 2-aminonaphthalenes can be regiospecifically sulfonated to a single specific sulfonation product when starting from the acylated amino compound of said aminonaphthalenes.

The process of the invention thus concerns the preparation of a sulfonated, sulfonyl-substituted, 2-amino or 2-acylamino naphthalene of the general formula (1)

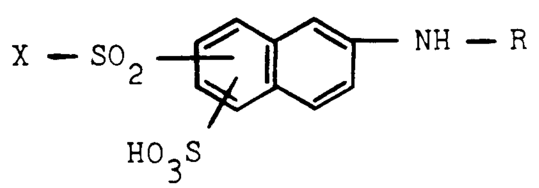

(1)

wherein:

- R is a hydrogen atom or a group of the general formula -CO-R' , in which
- R' is an alkyl, aryl or substituted alkyl or substituted aryl group,
- X is a group of the formula $-CH_2-CH_2-Z$ , in which Z is an organic or inorganic substituent capable of being split off by means of an alkaline agent, such as sodium hydroxide, and

the sulfo group $-SO_3H$ is in the 7-position if the group $X-SO_2-$ is in the 5-position, or the sulfo group is in the 5-position if the group $X-SO_2-$is in the 7-position,

by reacting a 2-acylamino naphthalene of the general formula (2)

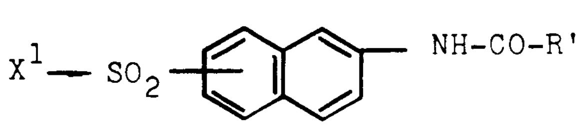

(2)

in which R' is defined as above, $X^1$ has one of the meanings of X or is preferably the $\beta$-hydroxyethyl group, and the group $X^1-SO_2-$ is in the 5- or 7- position, with a sulfonation agent, and optionally subsequent

deacetylation.

Preferably, R' is lower alkyl or 1 to 4 carbons, phenyl or substituted lower alkyl or substituted phenyl, the substituents of which may be one or more selected from halogen such as chlorine and bromine, nitro, alkoxy of 1 to 4 carbon atoms such as methoxy and ethoxy, phenoxy, alkyl or 1 to 4 carbon atoms such as methyl and ethyl (if substituted phenyl), and phenyl (if substituted alkyl). Preferably, the group $X^1$-$SO_2$- is $\beta$-chloro or $\beta$-bromoethylsulfonyl, $\beta$-hydroxyethylsulfonyl, $\beta$-sulfatoethylsulfonyl, $\beta$-phosphatoethylsulfonyl, $\beta$-thiosulfatoethylsulfonyl, most preferably $\beta$-hydroxyethylsulfonyl or $\beta$-sulfatoethylsulfonyl.

Common sulfonation agents can be used, such as fuming sulfuric acid, sulfur trioxide, chlorosulfonic acid.

Preferably the sulfonation is conducted in a sulfuric acid reaction medium with oleum (fuming sulfuric acid). The reaction product of the sulfonation can then be deacylated by heating the reaction product after it has been diluted with water, at a temperature of preferably about 100 to 115 ° C.

The compounds of formula (2) are prepared by acylation of amino-naphthalenes of the general formula (3)

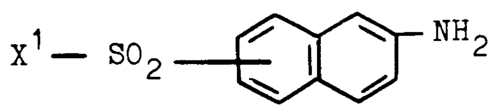 (3)

in which $X^1$ is defined as above and the group $X^1$-$SO_2$- is in the 5- or 7-position. The acylating agent which may be employed in the present process may be any of those currently utilized to acylate aromatic amines. Typical acylating agents are the anhydrides and acid halides such as compounds of the formula (4a) and (4b)

R' — CO-O-CO — R' (4a)

Y — OC — R' (4b)

wherein Y is halogen, preferably chlorine or bromine, and R′ is defined as above. Most preferably acetic anhydride, phthalic anhydride and benzoyl chloride are used.

The acylation reaction may be conducted in aqueous solution or in the solid phase, for example according to European Patent Publication No. 0,168,680A and US-Patent Specification 4,379,937.

The acylated, sulfonyl-substituted, aminonaphthalene is then sulfonated, preferably in sulfuric acid with oleum at a temperature between 0 and 80 ° C, preferably between 10 and 50 ° C, most preferably between 5 and 15 ° C.

Surprisingly, the regiospecificity of the process of this invention is not found to be present in the sulfonation of 2-amino-naphthalene sulfonic acids or 1-aminonaphthalene-5-or -6-$\beta$-(hydroxyethyl)-sulfones. Although acylation prior to sulfonation did result in some narrowing of the isomer distribution, this result was not found in all cases and an isomeric mixture was obtained in all instances. Thus, the regiospecificity of the process of the invention has been found to be present only in the 2-aminonaphthylamines containing the fiber reactive group $X^1$-$SO_2$- .

The following table illustrates the regiospecificity of the process of this invention:

| starting aminonaphthalene *) | sulfonation products (sulfo group introduced in ...-position) |
|---|---|
| 1a) 2-amino-naphthalene-7-VS | 1-sulfo (50 %)<br>5-sulfo (50 %) |
| 1b) 2-acetylamino-naphthalene-7-VS | 5-sulfo (100 %) |
| 2a) 2-amino-naphthalene-5-5-VS | 1-sulfo (50 %)<br>7-sulfo (50 %) |
| 2b) 2-acetylamino-naphthalene-5-VS | 7-sulfo (100 %) |

*) VS means the $\beta$-sulfatoethylsulfonyl group

As can be seen from the above table, the 2-amino-7-($\beta$-sulfatoethylsulfonyl)-naphthalene-5-sulfonic acid and 2-amino-5-($\beta$-sulfatoethylsulfonyl)-naphthalene-7-sulfonic acid can be prepared by the process of this invention as a single specific sulfonation product, i.e. in essentially pure form, free of other isomeric reaction products. This has great technical advantages, such as cost-savings and to provide a better starting material for the manufacture of azo dyestuffs since residual contamination with a sulfo-isomer can cause chromic shift in the resulting dyestuff.

Preferably, the sulfonation is conducted by dissolving the ($X^1$-$SO_2$-)-substituted N-acylated-2-aminonaphthalene compound of formula (2) in sulfuric acid at a temperature between 0 and 50 ° C, preferably between 10 and 20 ° C, to reduce the possibility of deacylating the reactant. The acylated compound is then sulfonated between 10 and 50 ° C, preferably between 10 and 20 ° C, by treatment with oleum.

After completing the sulfonation, the sulfonation product may be deacylated or recovered in the N-acyl form. Deacylation can be conducted by any known method, preferably by the addition of water to the reaction product and heating, for example, by dilution with water until the sulfuric acid concentration is between 85 and 100 percent, preferably 95 and 98%, and heating to 80 to 120 ° C, preferably 100 to 110 ° C.

It will be readily apparent to one skilled in the art that the $\beta$-sulfatoethylsulfonyl group may be replaced with another fiber reactive moiety represented by the general formula $-CH_2-CH_2-Z$ wherein Z is an inorganic or organic substituent which can be split off by the action of an alkaline reagent such as sodium hydroxide, sodium carbonate, etc. Replacement of the sulfato leaving group can be done either prior to or subsequent to sulfonation depending upon the reactivity of leaving group to the sulfonation step. Examples of other leaving substituents (Z) are halogen atoms, such as chlorine or bromine, an acyloxy group such as the acetyloxy group, a dialkylamino group such as the dimethylamino or diethylamino group, the thiosulfato or the phosphato group; preferably the leaving group is the sulfato group. Similarly, the fiber-reactive group may be converted into its vinyl form ($-CH=CH_2$) after the sulfo-substituted amino-naphthalene has been prepared. When the fiber-reactive group comprises the vinyl form in the following description, the designation $X^2$ is used.

While the 2-amino-7-($\beta$-sulfatoethylsulfonyl)-naphthalene-5-sulfonic acid prepared according to the invention, has not been found to be present in the art, the 2-amino-5-($\beta$-sulfatoethylsulfonyl)-naphthalene-7-sulfonic acid is known as a diazo component in a single anthraquinone-azo dye (see Table-Ex. 12 in German Offenlegungsschrift No. 2,840,120). Both bases, however, give most interesting azo dyestuffs with superior properties.

It is thus a further object of this invention to have found new and useful monoazo and disazo dyestuffs and their metal complexes from the following starting aminonaphthalenes according to the general formula (5)

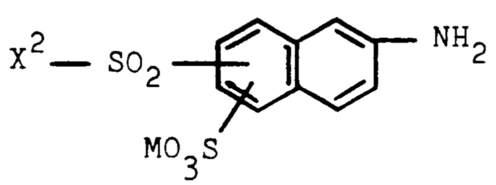

(5)

wherein M is a hydrogen atom or an alkali metal, such sodium, potassium or lithium, and $X^2$ has one of the meanings of X or is the vinyl group, and the substituent $X^2$-$SO_2$-is in the 5-position if the group $MO_3S$- is in the 7-position, or the substituent $X^2$-$SO_2$- is in the 7-position if the group $MO_3S$- is in the 5-position.

A further object of this invention are consequently the novel aminonaphthalene compounds according to the general (5A)

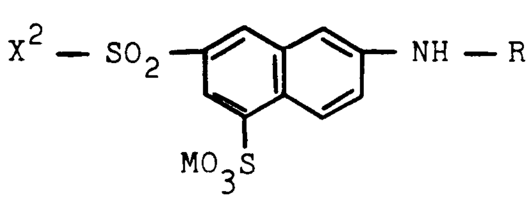

(5A)

wherein $X^2$, M and R are defined as above, R being preferably a hydrogen atom, the process for their preparation as described above, as well as their use as starting compounds, in particular as diazo components if R is hydrogen, for the synthesis of novel dyestuffs, in particular azo dyestuffs and their metal complex derivatives.

Novel monoazo dyestuffs according to the invention, containing the above diazotizable amines of formula (5) as diazo components, have the following general formula (6a)

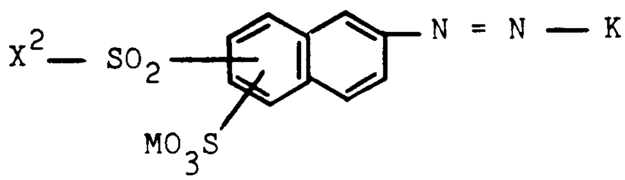

(6a)

wherein the ring positions for the $-SO_3M$ and $-SO_2-X^2$ groups are specified as given for formula (5), and K represents the residue of a coupling component of the phenol, amino-benzene, amino-naphthalene, naphthol, acetoacetic acid arylamide, pyrazolone or pyridone series. They are prepared by diazotization of an amino-naphthalene of the formula (5) with a coupling of formula H-K with K of the above meaning.

Novel metal-complex monoazo dyestuffs which correspond structurally to the dyestuffs of formula (6a), are in particular the 1:1-copper-, 1:2-chromium- and 1:2-cobalt-complex compounds of the monoazo compounds of the general formula (6A)

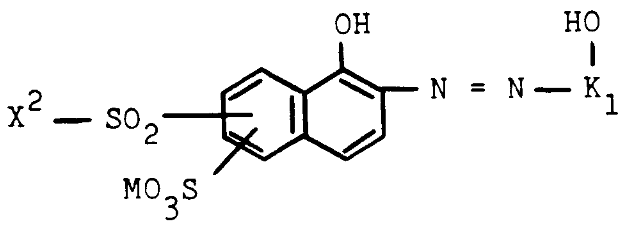

(6A)

in which M and $X^2$ and the positions of the groups $X^2-SO_2-$and $MO_3S-$ are defined as above for formula (6a) and $K_1$ is an unsubstituted or substituted moiety of the hydroxy-coupling component selected from the series mentioned above for the radical K, in which the hydroxy group is in the ortho-, respectively vicinal, position to the azo group, and in particular, the radical $-K_1-OH$ is a group of one of the formulae (7) shown below.

Preferred 1:1-copper-complex monoazo dyestuffs according to the invention, which correspond to the above-mentioned copper-complex compounds of formula (6A), are for example compounds according to the general formula (6B)

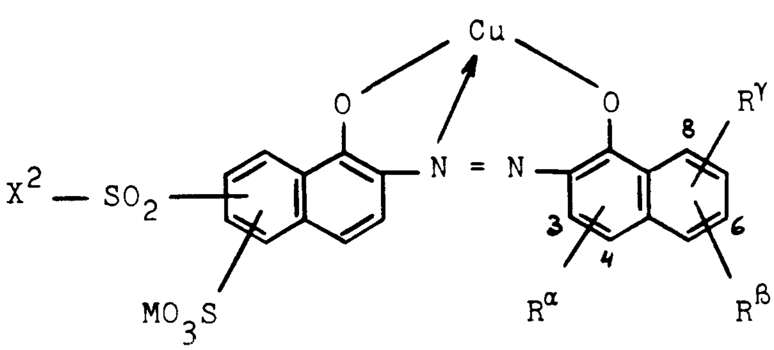

(6B)

wherein $X^2-SO_2-$ is in the 5- or 7-position and $MO_3S-$is in the 7- or 5-position, respectively, $R^\alpha$ is sulfo in the 4-position, and $R^\beta$ and $R^\gamma$ are both hydrogen, or $R^\alpha$ is sulfo in the 3-position, $R^\beta$ is sulfo in the 6-position and $R^\gamma$ is hydrogen, or $R^\alpha$ is sulfo in the 3-position, $R^\beta$ is sulfo in the 6-position and $R^\gamma$ is acetylamino in the

8-position, and $X^2$ and M have one of the above-mentioned, in particular preferred, meanings.

Diazotization is carried out by known methods, for ex. by the treatment of the amine with sodium nitrite in the presence of mineral acids or by treatment with nitrosyl sulfuric acid at a temperature of from -10 °C to +15 °C. Coupling reaction is carried out also in the usual manner found in the art, for ex. at a temperature of from 5 to 30 °C and, dependent from the coupling component - i.e. whether it is an amino or hydroxy coupler - in the range of from 2 to 8, such as from 2 to 5 or 4 to 8 (preferably 4 to 7), respectively.

Specific examples of the radical K of coupling components of formula H-K can be an acetoacetic acid anilide of the general formulae (7a)

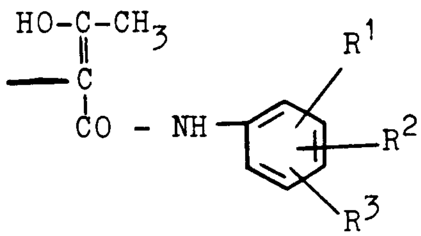

(7a)

wherein $R^1$, $R^2$ and $R^3$ have meanings which may be identical to or different from one another, and $R^1$ is hydrogen, hydroxy or a group $-SO_2X^2$ wherein $X^2$ has one of the above meanings, $R^2$ is hydrogen, chloro, bromo, alkyl of 1 to 4 carbon atoms, alkoxy of 1 to 4 carbon atoms or sulfo, and $R^3$ is hydrogen, chloro, alkyl or 1 to 4 carbon atoms, alkoxy of 1 to 4 carbon atoms or sulfo.

Further coupling components may be selected from substituted or unsubstituted $\alpha$- or $\beta$-naphthols, such as mono- and dihydroxy naphthalenes or amino-naphthols, the radical K of them having the following general formula (7b)

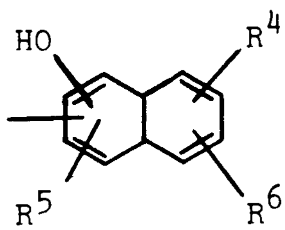

(7b)

wherein the free bond is in the ortho-position relative to the hydroxy group, $R^4$ hydrogen, alkyl of 1 to 4 carbon atoms, alkoxy of 1 to 4 carbon atoms, sulfo, amino, hydroxy, benzoylamino, sulfobenzoylamino, alkanoylamino of 2 to 5 carbon atoms, N-alkylamino of 1 to 4 carbon atoms or 3-phenylamino-5-chloro-s-triazin-1-yl-amino, the benzene moiety in the latter group being optionally substituted by sulfo, carboxy, chloro, methyl, ethyl, methoxy, ethoxy and/or nitro, preferably at least by a sulfo group, $R^5$ is hydrogen or sulfo and $R^6$ is hydrogen or sulfo, where $R^4$, $R^5$ and $R^6$ may have meanings which are identical to or different from another.

Further radicals -K of coupling components H-K are, for example, a radical of the general formula (7c)

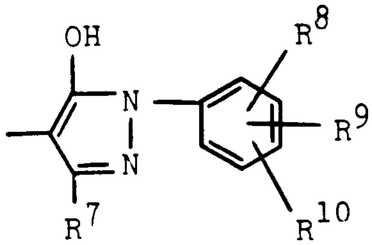

(7c)

in which $R^7$ is alkyl of 1 to 4 carbon atoms, such as methyl, alkoxy of 1 to 4 carbon atoms, carboxy or carbalkoxy of 2 to 5 carbon atoms, $R^8$ is hydrogen, sulfo, alkyl of 1 to 4 carbon atoms, alkoxy of 1 to 4 carbon atoms, chloro, bromo, hydroxy or $-SO_2-X^2$ , $R^9$ is hydrogen, sulfo, alkyl of 1 to 4 carbon atoms, alkoxy of 1 to 4 carbon atoms or chloro, and $R^{10}$ is hydrogen or sulfo.

Further radicals -K of coupling components H-K may be those of the general formula (7d)

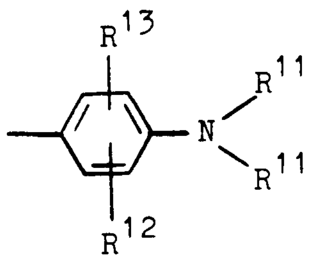

(7d)

wherein each $R^{11}$ is independently selected from hydrogen and alkyl of 1 to 4 carbon atoms unsubstituted or substituted by sulfo, carboxy, hydroxy, sulfato or alkoxy of 1 to 4 carbon atoms, $R^{12}$ is hydroxy, chloro, bromo alkyl of 1 to 4 carbon atoms, alkoxy of 1 to 4 carbon atoms, sulfo, alkylamino or 1 to 4 carbon atoms, alkanoylamino of 2 to 5 carbon atoms, ureido and $-SO_2-X^2$, and $R^{13}$ is hydrogen chloro, alkyl of 1 to 4 carbon atoms, alkoxy of 1 to 4 carbon atoms and sulfo.

Further radicals -K of coupling components H-K may be of the general formula (7e) or (7f)

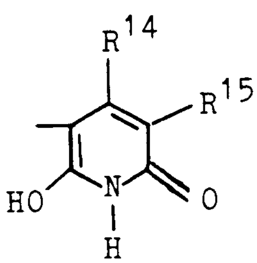

(7e)

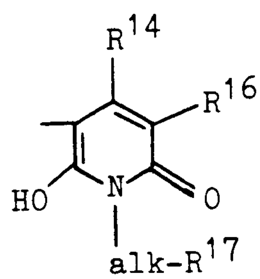

(7f)

in which

- $R^{14}$ is alkyl of 1 to 4 carbon atoms, such as methyl, phenyl or hydrogen,
- $R^{15}$ is hydrogen, chloro, bromo, sulfo, cyano, carbamoyl, carboxy or sulfamoyl, preferably hydrogen or carbamoyl,
- $R^{16}$ is hydrogen, chloro, bromo, sulfo, cyano, carbamoyl, carboxy or sulfamoyl, preferably hydrogen or carbamoyl,

and

- $R^{17}$ is hydroxy, sulfo, sulfato, amino, alkylamino of 1 to 4 carbon atoms, alkoxy of 1 to 4 carbon atoms and chloro, preferably methoxy or sulfo.

Further radicals -K of coupling components H-K are those of the general formula (7g)

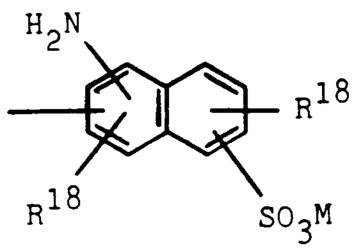

(7g)

in which the free bond is in the ortho- or para-position relatively to the amino group, and each $R^{18}$ is independently selected from hydrogen and sulfo, and M is defined as above.

Preferred coupling components in the monoazo dyestuffs of the invention are 1-acetoacetylamino-3-methyl-6-methoxy-benzene-4-sulfonic acid, 1-acetoacetylamino-3,6-dimethoxy-benzene-4-sulfonic acid, 1 hydroxynaphthalene-3,6-disulfonic acid, 1-hydroxynaphthalene-4-sulfonic acid, 1-acetylamino-8-hydroxy-naphthalene-3,6-disulfonic acid, 1-acetylamino-8-hydroxy-naphthalene-4,6-disulfonic acid, 1-benzoylamino-8-hydroxy-naphthalene-3,6-disulfonic acid, 1-benzoylamino-8-hydroxy-naphthalene-4,6-disulfonic acid, 2-acetylamino-5-hydroxy-naphthalene-7-sulfonic acid, 3-acetylamino-5-hydroxy-naphthalene-7-sulfonic acid, 1-(4'-sulfophenyl)-3-methyl-5-pyrazolone, 1-(4'-sulfophenyl)-3-carboxy-5-pyrazolone, 1-[4'-(2''-sulfatoethylsulfonyl)-phenyl]-3-methyl-5-pyrazolone, N,N-bis-(2'-sulfatoethyl)-3-chloro-aniline, N-(2'-sulfatoethyl)-4-methyl-6-hydroxy-pyridone, and 1-hydroxy-3,6-disulfo-8-[5'-chloro-3'-(2'' or 3''- or 4''-sulfophenyl)-amino-2',4',6'-triazin-1'-yl]-amino-naphthalene.

Most preferred coupling components are 1-acetoacetylamino-3-methyl-6-methoxy-benzene-4-sulfonic acid, 1,-hydroxy-naphthalene-3,6-disulfonic acid, 1-hydroxy-naphthalene-4-sulfonic acid, 1-acetylamino-8-hydroxy-naphthalene-3,6-disulfonic acid, 1-acetylamino-8-hydroxy-naphthalene-4,6-disulfonic acid, 1-benzoylamino-8-hydroxy-naphthalene-3,6-disulfonic acid, 1-benzoylamino-8-hydroxy-naphthalene-4,6-disulfonic acid, 2-acetylamino-5-hydroxy-naphthalene-7-sulfonic acid and 1-hydroxy-8-[5'-chloro-3'-(2''or 3''-or 4''-sulfophenyl)-amino-2',4',6'-triazin-1'-yl]-amino-naphthalene-3,6-disulfonic acid.

The naphthylamine bases of general formula (5) are also useful in the preparation of novel fiber reactive disazo dyestuffs and their metal complexes, for ex. of novel disazo compounds according corresponding to the general formula (6b)

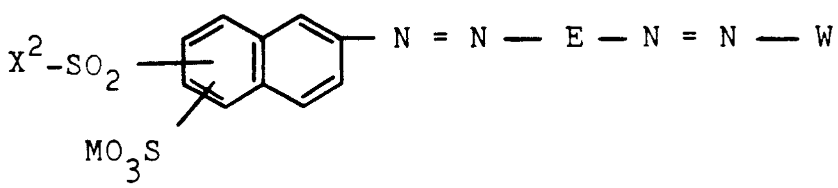

(6b)

in which

M is defined as above, the $X^2$-$SO_2$- and $MO_3S$- groups are in the positions as mentioned above for formula (5),

W is a radical of a coupling component H-K defined above, or a diazo component D,

D representing a group of the formula (8)

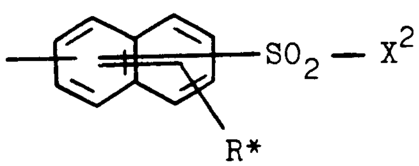

(8)

in which R* is hydrogen or sulfo, and the sulfo group and the group-$SO_2$-$X^2$ are preferably in the positions as mentioned for formula (5) and in particular for formula (5A), or representing phenyl or naphthyl, each unsubstituted or substituted by one to three substituents independently selected from chloro, fluoro, bromo, sulfo, carboxy, alkoxy of 1 to 4 carbon atoms, alkyl of 1 to 4 carbon atoms, benzoylamino, sulfobenzoylamino, alkanoylamino of 2 to 5 carbon atoms, N-alkylamino of 1 to 4 carbon atoms, N,N-dialkylamino of 1 to 4 carbon atoms in each alkyl and 3-phenylamino-5-chloro-s-triazin-1-ylamino, the benzene moiety in the latter group being optionally substituted by sulfo, carboxy, chloro, methyl, ethyl, methoxy, ethoxy and/or nitro, preferably at least by sulfo group, and unsubstituted or substituted by one or two groups -$SO_2$-$X^2$ defined above, and

E represents a 1,4-phenylene or 1,4-naphthylene optionally substituted with 1 or 2 substituents independently selected from chloro, fluoro, bromo, sulfo, carboxy, alkoxy of 1 to 4 carbon atoms, alkyl of 1 to 4 carbon atoms, benzoylamino, sulfobenzoylamino, alkanoylamino of 2 to 5 carbon atoms, ureido and N,N-dialkylamino of 1 to 4 carbon atoms in each alkyl, or E represents a 2,6- or 2,7-naphthylene substituted in the 1-position by hydroxy and substituted by 1 or 2 sulfo groups, or is 1-hydroxy-8-amino-3,6- or -4,6-disulfo-naphth-2,7-ylene.

If the disazo dyes of formula (6b) represent metal-complexes such as 1:1-copper-, 1:2-cobalt- or 1:2-chromium-complex dyes, the radical E, if it is 1,4-phenylene or 1,4-naphthylene, is additionally substituted

by a hydroxy group in the ortho-position relative to one of the azo groups and a diazo component is optionally also substituted by a hydroxy group in the ortho-position relatively to the azo groups, and the metal is bonded to the hydroxy groups of the diazo and coupling components or of E and of the diazo or the coupling component.

The diazo components of formula $H_2N$-D are known in the art, respectively can be prepared by known methods (see, for example, the US-PS 2,657,205 and 4,492,654 which are hereby incorporated by reference). The preparation of such disazo dyestuffs is fully explained in these Specifications (see specifically Columns 3 and 4 of US-PS 4,492,654).

The disazo compounds of formula (6b) in which W is D may be prepared by diazotization of one of the starting aromatic amine of formula (5) or of formula $H_2N$-D by known procedures, for example, in acidic, aqueous medium with sodium nitrite at a temperature between -5 °C and +15 °C, and then coupled to an aromatic compound represented by the general formula H-E-H wherein E is defined as above. The first coupling is usually conducted under acidic conditions at pH from about 1 to about 3 at a temperature of about 5 to 30 °C. The second coupling is then effected at a higher pH in the range of 4 to 8, preferably 5 to 7, and at a temperature of about 5 to 30 °C.

The disazo compounds of formula (6b) in which W is K, may be prepared by diazotization of the starting amino compound of formula (9)

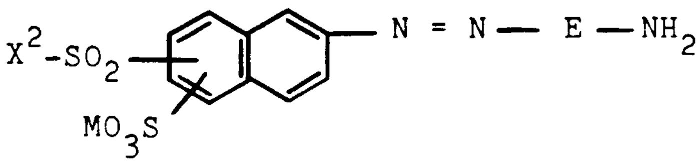

(9)

and coupling with a compound of formula H-K according to a procedure as mentioned above for the monoazo dyestuffs.

Examples of aromatic amines of formula D-$NH_2$ are 2-bromo-4-(β-sulfatoethylsulfonyl)-aniline, 2-fluoro-aniline, 2-bromo-aniline, 2-methoxy-5-methyl-4-(β-sulfatoethylsulfonyl)-aniline, 3-(β-sulfatoethylsulfonyl)-aniline, 2-chloro-4-(β-sulfatoethylsulfonyl)-aniline, 2-nitro-4-(β-sulfatoethylsulfonyl)-aniline, 2-sulfo-4-(β-sulfatoethylsulfonyl)-aniline, 4-vinylsulfonyl-aniline, 4-(β-chloroethylsulfonyl)-aniline, 4-(β-thiosulfatoethylsulfonyl)-aniline, 4(β-acetoxyethylsulfonyl)-aniline, 4(β-benozyloxyethylsulfonyl)-aniline, 2-methoxy-5-(β-sulfatoethylsulfonyl)-aniline, 2-chloro-aniline, 2,4-dichloro-aniline, 4-aminobenzoic acid, aniline-2-sulfonic acid, aniline-3-sulfonic acid, aniline-4-sulfonic acid, aniline-2,4-disulfonic acid, aniline-2,5-disulfonic acid, 2-amino-naphthalene-1,5-disulfonic acid, 2-chloroaniline-5-sulfonic acid, 4-(β-sulfatoethylsulfonyl)-aniline, 3-(β-sulfatoethylsulfonyl)-4-methoxy-aniline, 2,5-dimethoxy-4-(β-sulfatoethylsulfonyl)-aniline, 4-(β-sulfatoethylsulfonyl)-1-amino-naphthalene, 6-(β-sulfatoethylsulfonyl)-2-amino-naphthalene-8-sulfonic acid, 8-(β-sulfatoethylsulfonyl)-2-amino-naphthalene, 8-(β-sulfato-ethylsulfonyl)-2-amino-naphthalene-6-sulfonic acid, 6-(β-sulfatoethylsulfonyl)-2-amino-naphthalene, 6-(β-sulfato-ethylsulfonyl)-2-amino-naphthalene-1-sulfonic acid and 1-sulfo-4-[5'-chloro-3'-(2''- or 3'' or 4''-sulfophenyl)-amino-2',4',6'-triazin-1'-yl]-amino-2-amino-benzene.

Exemplary divalent couplers of formula H-E-H are 1-amino-8-naphthol-3,6-disulfonic acid, 1,8-dihydroxynaphthalene-3,6-sulfonic acid, 1,3-dihydroxybenzene and 1-amino-8-naphthol-4,6-disulfonic acid.

The metal-complex dyes, such as copper, nickel, chromium and cobalt metal complexes, of the dyes of general formula (6a), respectively (6A), and (6b) can be prepared by known methods from the o,o'-dihydroxy-azo dye or the reactive equivalent thereof, e.g., o-alkoxy-o'-hydroxy-azo dyes, or by treating an o'-hydroxy-azo dye which contains a hydrogen atom in the ortho-position to the azo group, such as the metal free monoazo or disazo compounds of formula (6a) and (6b), with a metal such as copper, or a metal-yielding agent, such as copper-yielding agent, and an oxidizing agent, such as hydrogen peroxide, in a weakly acid solution. U.S. Patent Specification 4,400,317 for instance describes a method of preparing the metal complex by treatment with non-salt forming metals. The metal-complex dyes of the invention may contain on complexed metal atom to one dye molecule or one metal atom to two molecules. In the case of chromium or cobalt complexes the ratio of metal atoms to dye molecules is preferably 1:2 respectively and in the case of copper and nickel the ratio is 1:1 respectively.

The dyestuffs of the invention can be separated from the reaction medium after their preparation by known methods suitable for water-soluble compounds, such as, by precipitation from the reaction medium with electrolytes, such as sodium chloride or potassium chloride, or by evaporation of the reaction medium, for example by spray-drying. When the latter method for isolation is chosen, it can be advantageous to remove sulfates if they are present in the synthesis solutions, before evaporation. This can be done by

precipitation of the sulfates as calcium sulfate and filtration. In some cases, it is also possible for the solutions obtained in the synthesis to be used directly, after standardization with water, as a liquid dyestuff composition. These liquid dyestuff compositions, especially useful in chemical-pad-steam applications, show no chemical or physical changes on long term storage.

The dyestuffs according to the invention are suitable, as water-soluble dyestuffs, for coloring (dyeing and printing) of fibers, leather or other materials containing hydroxy groups and/or amino groups. They may be used in their free acid form, preferably in form of their salts. Exemplary materials are natural, regenerated or synthetic, nitrogen-containing fibers and natural, regenerated or synthetic hydroxy group containing fibers. The dyestuffs according to the invention are capable of coloring these materials to deep, brilliant shades ranging from yellow to blue with superior properties. The fiber-reactive groups in the compounds of the invention can react with the $NH_2$- and OH-groups of the material, such as cotton containing hydroxy groups, to form a covalent bond and thus form a bonded link with the fiber.

The present invention also relates to the use of the dyestuffs of the invention for coloring (such as dyeing and printing) such materials and to a process for coloring such materials. This process comprises contacting a dyestuff of the instant invention, preferably in the form of an aqueous solution, with the material and fixing said dyestuff on it optionally under the action of an alkaline agent and/or heat.

Typical nitrogen-containing synthetic materials useful in this invention are polyurethanes and polyamides, such as nylon-6, nylon-6/6 and nylon-11. Typical natural polyamide materials are silk and wool and other animal hair products. Typical materials containing hydroxy groups are polyvinyl alcohols, cellulosic materials such as cotton, other vegetable fibers, such as linen, hemp, jute and their regenerated products, such as viscose rayon or cuprammonium rayon.

The novel azo dyestuffs and their metal complexes, according to the invention, can be applied by the known application techniques for fiber-reactive dyestuffs. In general, a procedure is followed in which an aqueous solution of the compounds of their metal complexes are applied to the materials, optionally in the presence of a thickener and/or other auxiliaries to improve the affinity, levelling and migration properties. After application the dyestuff is then fixed to the fiber.

The dyestuffs of the invention are applied to natural or regenerated or synthetic polyamide fibers of polyurethane fibers or to leather by conventional techniques from an aqueous acid to aqueous neutral solution (pH range from about 3 to 6.5), usually by the exhaustion method, and are fixed on these fibers by the application of heat at a temperature between 60 and 130 °C. It is possible for example to add acetic acid or acetic acid and ammonium acetate as a buffer to the bath containing the dyestuffs in order to obtain the desired pH value. Addition of leveling agents, for example those based on a reaction product of cyanuric chloride with three moles of an aminobenzenesulfonic acid and/or an amino-naphthalenesulfonic acid of those based on a reaction product of stearylamine and ethylene oxide, can be used for the purpose of achieving level dyeings. The compounds of the invention can be applied and fixed on the material by the exhaustion process either at the boiling point or at a higher temperature, for example, at 105 to 120 °C, under pressure. It is expedient for the dyeing to be started with a slow increase in temperature to 60 °C and, after some time, for the temperature to be increased slowly to a higher temperature.

In the coloring of fiber materials containing hydroxy groups, the dyestuff of the invention is generally applied to the fiber from a weakly acid to alkaline solution and fixed on the fiber by an alkaline agent subsequently added to the dye bath or applied directly to the fiber. Typical alkaline substances which can be used in these fixing solutions are sodium hydroxide, sodium carbonate, sodium bicarbonate, potassium hydroxide, potassium carbonate, trisodium phosphate or sodium or potassium silicate or waterglass. The dyestuffs can be applied by exhaustion dyeing procedures or the chemical pad steam process. In the exhaust method, the fiber material is treated in an aqueous-alkaline solution of the compound of the invention, preferably in the presence of an electrolyte, such as sodium chloride or sodium sulfate, at an elevated temperature between 30 and 130 °C. It is preferably for the dyeing to be started at a low temperature and for the temperature of the exhaustion bath to be slowly increased to about 60 to 130 °C, and completing the fixation step in this temperature range.

In the chemical-pad-steam method, the fabric is continuously passed through a dyebath, dried, passed through an alkaline chemical pad, fixed by steaming, washed an dried. Fiber reactive dyes perform well in this application.

If the dyestuffs of the invention are applied to the fiber material in the form of printing pastes, it is usual to employ thickeners, such as sodium alginate, cellulose ether, tragacanth or gum arabic, optionally with the addition of a printing auxiliary and an alkaline compound. These prints are then treated with hot air at a temperature between 70 and 230 °C, preferably between 100 and 150 °C (thermofixes), or steamed. The dyestuffs of invention can be applied to the fiber by customary printing processes such as a one-step procedure using a printing paste of the dyestuff containing sodium bicarbonate or one of the other alkaline

agents with subsequent fixation of the dye by steaming at 101 to 103°C. The also can be applied to the fiber by a two-step process of applying a neutral or weakly acid printing paste of the dyestuff and then fixing it either by passing the printed material through a hot alkaline bath containing electrolytes. Alternatively, it can be overpadded with an alkaline liquor containing electrolytes and left to stand at room temperature but usually it is treated with heat using hot steam or hot air. If an electrolyte-containing alkaline bath is used for fixing, the bath temperature is 60 to 105°C, so that subsequent treatment by hot air or steam can be eleminated.

If the material is impregnated with the dyestuff of the invention and treated with a strong aqueous alkali (e.g. sodium hydroxide or potassium hydroxide and/or sodium silicate or potassium silicate or trisodium phosphate), it is sufficient for the moist goods (usually prints) to be left to stand at room temperature for a relatively long period to fix the dyestuff. The colored materials thus obtained are then after-treated, rinsed and dried, in the usual manner.

The following Examples illustrate the invention. Parts and percentages are by weight unless stated otherwise. Parts by weight relate to parts by volume as the kilogram relates to the liter.

The azo compounds described are in general specified in the form of the free acids; in general, they are prepared and isolated in the form of their sodium or potassium salts and used for dyeing in the form of their salts. The starting compounds mentioned in the form of the free acid are employed in the synthesis, as such or in the form of their salts preferably alkali metal salts, such as sodium or potassium salts.

The $\lambda_{max}$-values given in the Examples for the azo compounds according to the invention represent the absorption maxima in the visible region, measured on the alkali metal salts of the dyestuffs according to the invention in aqeuous solution.

**Example 1**

This example illustrates the preparation of the novel 2-amino-7-($\beta$-sulfatoethylsulfonyl)-naphthalene-5-sulfonic acid and its diazonium salt.
Into a 250 ml round bottom flask equipped with a mechanical stirrer, thermometer and drying tube were charged 32.0 grams of sulfuric acid monohydrate at a temperature of 10°C. Then 14.65 grams (= 0,05 mole) of 2-N-acetylamino-naphthalene-7-$\beta$-hydroxyethylsulfone were slowly added over a two hour period. The reaction mixture was then stirred at 10°C for one additional hour and then 20.0 grams of 65 % oleum were added. After completing the oleum addition, the reaction mixture was allowed to warm to room temperature at which point the sulfonation reaction was complete.

3.0 grams of ice were added to the reaction mixture and it was heated to 100-110°C for three hours to effect deacetylation. The reaction mixture was then cooled to 15°C, and 15.8 grams of a 40 % nitrosyl sulfuric acid solution was added to effect diazotization. The reaction mixture was stirred two hours at 10°C and then drowned onto a mixture of ice and water. The produced 2-diazo-naphthalene-7-($\beta$-sulfatoethylsulfone)-5-sulfonic acid was totally water-soluble, and the solution was saved for future use in preparing fibre-reacting dyestuffs.

**Example 2**

This example illustrates the preparation of 2-amino-5-($\beta$-sulfatoethylsulfonyl)-naphthalene-7-sulfonic acid. Into 250 ml round bottom flask equipped with a mechanical stirrer, thermometer and drying tube were charged 82.0 grams of sulfuric acid monohydrate at a temperature of approximately 10°C. Then 36.9 grams of 2-N-acetylamino-naphthalene-5-$\beta$-hydroxyethylsulfone (0.125 mole) were slowly added over a two hour period. The reaction mixture was then stirred at about 10°C for one additional hour and then 50.0 grams of 65 % oleum were added. After completing the oleum addition, the reaction mixture was allowed to warm at room temperature at which point the sulfonation reaction was complete.

Approximately 7.2 grams of ice were added to the reaction mixture and it was then heated to 100-110°C for two hours to effect deacetylation. The reaction mixture was then cooled to approximately 20°C and added to a mixture of 125 grams of water, 125 grams of sodium chloride and ice in an amount sufficient to maintain the temperature at 0°C. The product was totally water-soluble at this point. The pH was adjusted to 4.0-4.5 with 180.0 grams of sodium carbonate, cooled to 10°C and filtered to remove the precipitated sodium sulfate. The resulting solution which contained the 2-amino-5-($\beta$-sulfatoethylsulfonyl)-naphthalene-7-sulfonic acid was retained for future use.

**Example 3**

A dyestuff of the following formula in its free acid form was prepared:

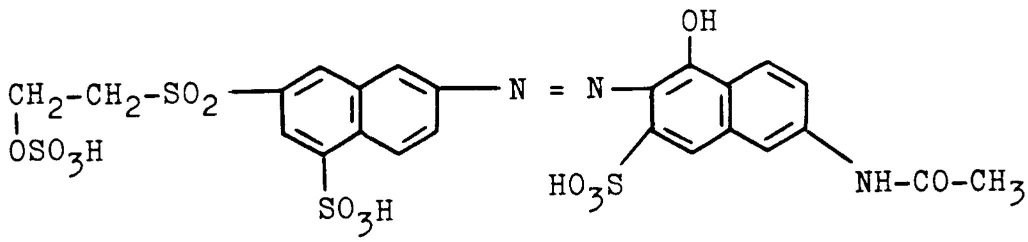

($\lambda_{max}$ = 490 nm).

To the diazo compound prepared in Example 1 was added 14.0 grams (0.05 mole) of 3-acetylamino-8-hydroxynaphthalene-6-sulfonic acid. The pH of the reaction mixture was adjusted to 4.0-4.5 by the addition of sodium carbonate. The reaction mixture was stirred until the coupling reaction was complete, cooled to 0-5 ° C and filtered to remove the precipitated sodium sulfate. The resulting solution was dried at 60 ° C under reduced pressure to yield an orange, electrolyte-salt containing powder of the above dyestuff of the invention.

This dyestuff of the invention produces when applied according to the known dyeing methods for fibre-reactive dyestuffs, deep orange shades on cotton having good fastness properties; it is particular suitable for use in combination with other fibre-reactive dyes, i.e. in a dichromatic or trichromatic dyeing system.

**Example 4**

A dyestuff of the following formula in its free acid form was prepared:

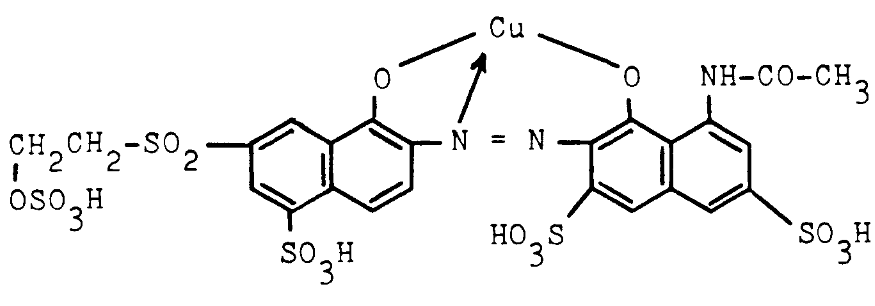

($\lambda_{max}$ = 542 nm).

To the diazo compound prepared in Example 1 was added 18.0 grams (0.05 mole) of 1-hydroxy-8-acetylamino-naphthalene-3,6-disulfonic acid. The pH of the reaction mixture was adjusted to 4.0-4.5 by the addition of sodium carbonate; it was stirred at a pH of 4.5-5.0 until the coupling procedure was complete. The solution was cooled to 0-5 ° C and filtered to remove the precipitated sodium sulfate. To the resulting solution was added 12.5 grams of copper sulfate pentahydrate and, at a pH of 4.5-5.0 and a temperature of 40-45 ° C, 51.0 grams of an aqueous 10 % hydrogen peroxide solution over one hour. Sodium carbonate was used to maintain the pH of the reaction. When the copperization was completed, 80.0 grams of potassium chloride were added, the reaction mixture was stirred over night, filtered, and the resulting press cake dried at 50 ° C under reduced pressure. A blue electrolyte-containing powder of the above-mentioned copper-complex dyestuff according to the invention was obtained which dyes cotton, according to usual application methods for fibre-reactive dyestuffs, in deep blue shades having food fastness properties; the dyestuffs of the invention is particular suitable for use in combination with other fibre-reactive dyes, i.e. in a dichromatic or trichromatic dyeing system.

**Example 5**

A dyestuff of the following formula in its free acid form was prepared:

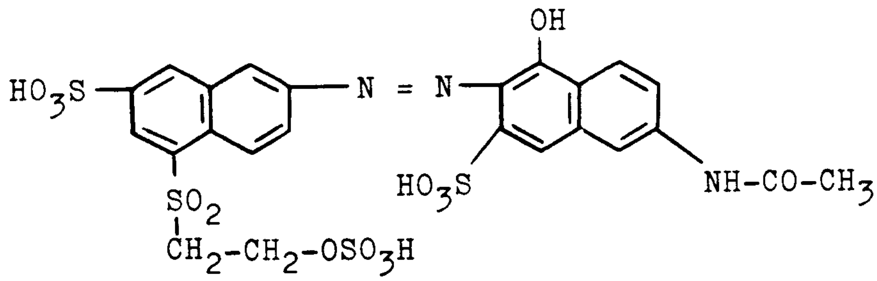

($\lambda_{max}$ = 488 nm).

Into a 2 liter beaker equipped with a mechanical stirrer, thermometer and pH probe was added a solution prepared according to Example 2, containing 82.2 grams of 2-amino-7-sulfo-naphthalene-5-(β-sulfatoethyl)-sulfone, and 40.0 grams of sulfuric acid. While maintaining the temperature of 0-5 ° C and pH less than 2, 35 grams of a 40 % solution of sodium nitrite were added over one hour. The reaction was stirred for one hour with a slight excess of nitrite present. The excess nitrite was decomposed by the addition of 1 gram of sulfamic acid.

756 grams of a solution containing 56.2 grams of 3-acetamino-8-hydroxy-naphthalene-6-sulfonic acid were added to the reaction mixture and the pH was adjusted to 5.0-5.5 by the addition of 142 grams of 15 % sodium carbonate solution. The resulting solution was dried to yield an orange dyestuff which produces deep orange fast shades on cotton according to usual application and fixation methods known for fibre-reactive dyes; it is suitable particular for use in combination with other fibre-reactive dyes, i.e. in a dichromatic or trichromatic dyeing system.

**Example 6**

A dyestuff of the following formula in its free acid form was prepared:

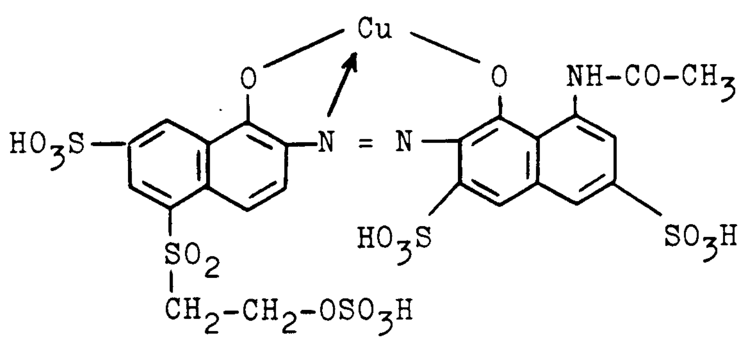

($\lambda_{max}$ = 540 nm).

Into a 4 liter flask equipped with a mechanical stirrer, thermometer and pH probe was added a solution prepared according to Example 2, containing 82.2 grams of 2-amino-7-sulfo-naphthalene-5-(β-sulfatoethyl)-sulfone, and 40.0 grams of sulfuric acid. While maintaining the temperature at 0-5 ° C and pH less than 2, 35 grams of a 40 % solution of sodium nitrite were added over one hour. The reaction was stirred for one hour with a slight excess of nitrite present. The excess nitrite was decomposed by the addition of 1 gram of sulfamic acid.

442 grams of a solution containing 72.2 grams of 1-acetamino-8-hydroxy-naphthalene-3,6-disulfonic acid were added to the pH of the reaction adjusted to 5.0-5.5 by the addition of 105 grams of a 15 % solution of

sodium carbonate. Then 250 grams of a 20% solution of copper sulfate pentahydrate were added and over 2 hours, 100 grams of 30 % hydrogen peroxide were added while maintaining the pH at 5.0-5.5 by the addition of 15 grams of sodium carbonate.

250 grams of potassium chloride were added, the reaction stirred overnight and filtered. The resulting presscake was dried to a blue dyestuff which produces deep blue fast shades on cotton according to usual application and fixation methods known for fibre-reactive dyes; it is suitable particular for use in combination with other fibre-reactive dyes, i.e. in a dichromatic or trichromatic dyeing system.

**Example 7**

A dyestuff of the following formula in its free acid form was prepared:

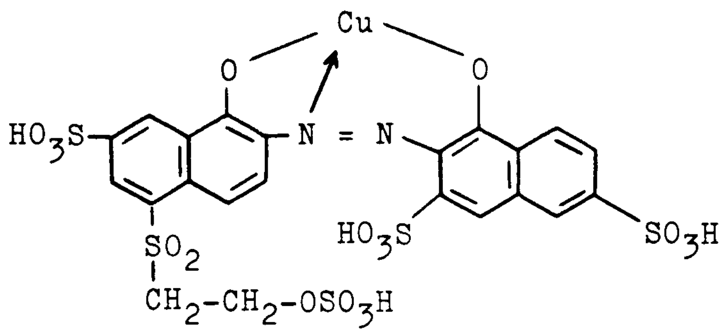

($\lambda_{max}$ = 532 nm).

This dyestuff was prepared substantially in accordance with the procedure of Example 4 or 6 except the coupling component used was 1-hydroxy-naphthalene-3,6-disulfonic acid. The resulting dyestuff produces deep, blue fast shades on cotton according to usual application and fixation methods known for fibre-reactive dyes; it is suitable particular for use in combination with other fibre-reactive dyes, i.e. in a dichromatic or trichromatic dyeing system.

**Example 8**

A dyestuff of the following formula in its free acid form was prepared:

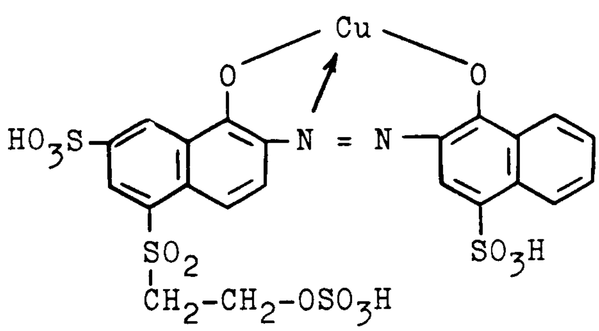

($\lambda_{max}$ = 529 nm).

This dyestuff was prepared substantially in accordance with the procedure of Example 4 or 6 except the coupling component was 1-hydroxy-naphthalene-4-sulfonic acid. The resulting dyestuff produces deep, blue fast shades on cotton according to usual application and fixation methods known for fibre-reactive dyes; it is suitable particular for use in combination with other fibre-reactive dyes, i.e. in a dichromatic or trichromatic dyeing system.

**Example 9**

A dyestuff of the following formula in its free acid form was prepared:

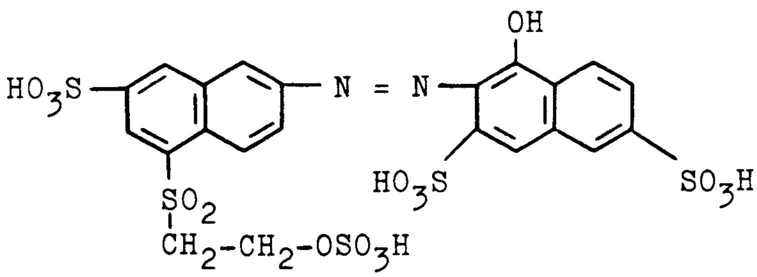

($\lambda_{max}$ = 492 nm).

This dyestuff was prepared substantially in accordance with Example 7 except the coppering step was not conducted. The resulting dyestuff produces a deep, scarlet fast shade on cotton according to usual application and fixation methods known for fibre-reactive dyes; it is suitable particular for use in combination with other fibre-active dyes, i.e. in a dichromatic or trichromatic dyeing system.

**Example 10**

A dyestuff of the following formula in its free acid form was prepared:

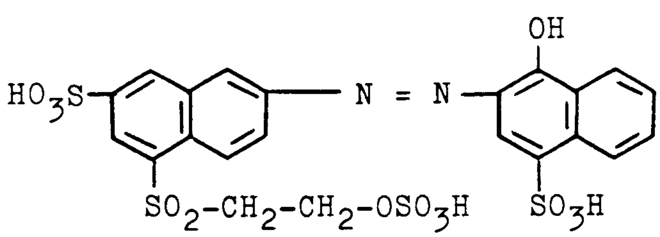

($\lambda_{max}$ = 492 nm).

This dyestuff was prepared in accordance with the procedure of Example 8 except the coppering step was not done. The resulting dyestuff produces deep, scarlet shades on cotton having good fastness properties, according to usual application and fixation methods known for fibre-reactive dyes; it is suitable particular for use in combination with other fibre-reactive dyes, i.e. in a dichromatic or trichromatic dyeing system.

**Example 11**

A dyestuff of the following formula in its free acid form was prepared:

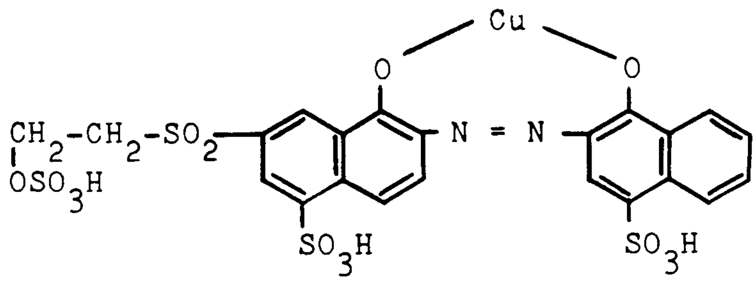

$(\lambda_{max} = 530\ nm)$.

This dyestuff was prepared substantially in accordance with the procedure of Example 4 except the coupling component was 1-hydroxy-naphthalene-4-sulfonic acid. The resulting dyestuff produced deep, blue shades on cotton which showed good fastness properties, when applied according to dyeing and printing methods known for fibre-reactive dyestuffs.

**Example 12**

A dyestuff of the following formula in its free acid form was prepared:

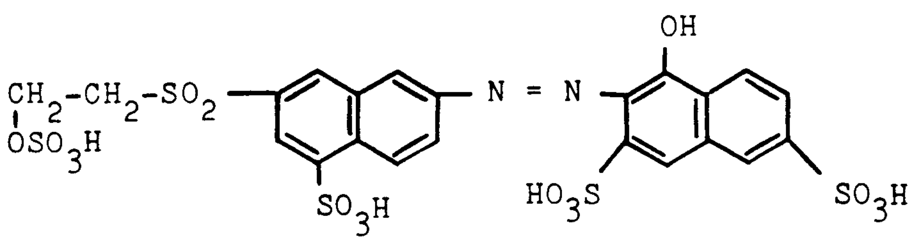

$(\lambda_{max} = 495\ nm)$.

To a 0.05 mole diazo solution prepared according to Example 1, was added 15.25 grams of 1-hydroxy-naphthalene-3,6-disulfonic acid. The pH was adjusted to 4.0-4.5, and the reaction mixture was stirred until the coupling was complete, thereafter cooled to 0-5 ° C and filtered to remove the precipitated sodium sulfate. To the resulting solution was added 82 grams of potassium chloride, and the reaction mixture was stirred over night and then filtered. The resulting press cake was dried under reduced pressure at 50 ° C. The dyestuff according to the invention of the above formula, obtained in form of its potassium salt, dyes cotton in deep orange shades with good fastness properties, when applied according to dyeing at printing methods known for fiber-reactive dyestuffs.

**Example 13**

A dyestuff of the following formula in its free acid form was prepared:

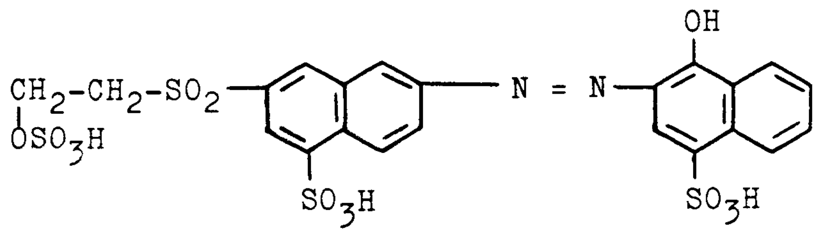

($\lambda_{max}$ = 495 nm).

To a 0.05 mole diazo solution prepared according to Example 1, was added 11.2 grams (0.05 moles) of 1-hydroxy-naphthalene-4-sulfonic acid. The pH was adjusted to 4.0-4.5 using sodium carbonate, and the reaction mixture was stirred until coupling was complete. It was then cooled to a temperature of 0-5 °C and filtered to remove the precipitated sodium sulfate. To the filtrate was added 85 grams of potassium chloride, the whole was stirred over night and then filtered. The resulting press cake was dried under reduced pressure at 60 °C. The dyestuff of the invention obtained in form of its potassium salt, produces on cotton, according to the usual dyeing and printing methods for fibre-reactive dyestuffs, deep orange shades with food fastness properties.

**Example 14**

A dyestuff of the following formula in its free acid form was prepared:

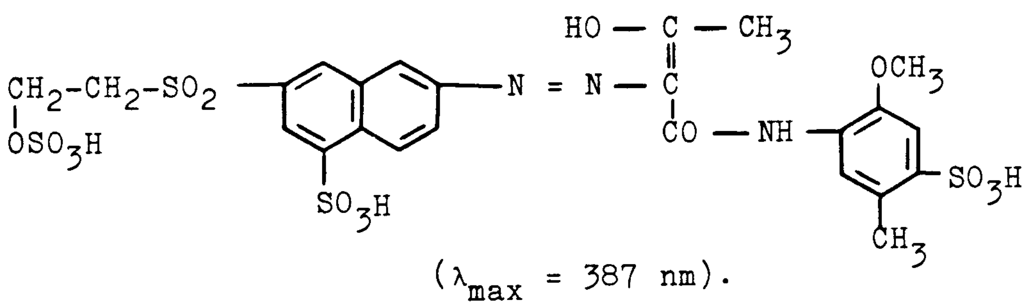

($\lambda_{max}$ = 387 nm).

To an aqueous solution containing 15.9 grams of N-acetoacetyl-(2-methoxy-5-methyl-4-sulfo)-aniline in the form of its ammonium salt (corresponding to 0.05 mole) was added the diazo solution prepared in Example 1, in the equivalent amount of 0.05 moles relative to the starting naphthylamine compound. The pH during the addition was maintained at 4.0-4.5 by means of sodium carbonate. The resulting solution was then cooled to a temperature of 0-5 °C, filtered to remove the precipitated sodium sulfate and dried under reduced pressure at 60 °C. A yellow powder of the sodium salt of the above showed dyestuff was obtained which dyes cotton, according to the usual application and fixation methods for fibre-reactive dyestuffs, in deep yellow shades with good fastness properties.

**Example 15**

A dyestuff of the following formula in its free acid form was prepared:

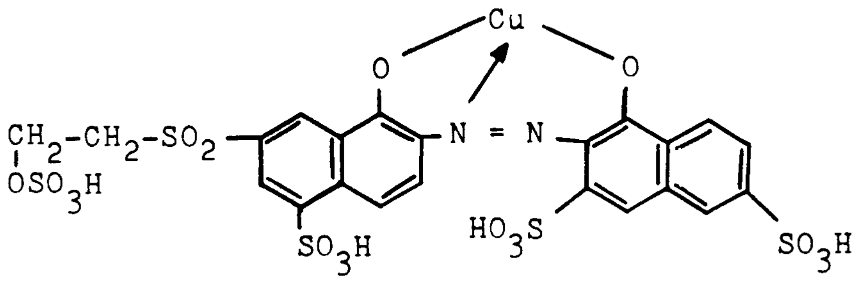

($\lambda_{max}$ = 534 nm)

substantially in accordance with the procedure of Example 4 except that the coupling component was 1-hydroxy-napthalene-3,6-disulfonic acid. The dyestuffs according to the invention obtained produces on cotton, according to the usual dyeing and printing methods for fibre-reactive dyestuffs, dyeings and prints in deep blue shades with good fastnesses.

Examples 16 to 49

Other useful dyestuffs with similar dyeing properties can be obtained when in the above examples the 2-amino-7-($\beta$-sulfatoethylsulfonyl)-naphthalene-5-sulfonic acid or the 2-amino-5-($\beta$-sulfatoethylsulfonyl)-naphthalene-7-sulfonic acids is coupled with a coupling component set forth in the following Table.

| Bsp. | Naphthylamine Base | Coupler | Shade |
|---|---|---|---|
| 16 | 2-amino-5-(ß-sulfatoethylsulfonyl)-naphthalene-7-sulfonic acid | 1-acetoacetylamino-3-methyl-6-methoxybenzene-4-sulfonic acid | yellow |
| 17 | dito | 1-acetoacetylamino-3,6-dimethoxy-benzene-4-sulfonic acid | yellow |
| 18 | dito | 1-hydroxynaphthalene-3,6-disulfonic acid | orange |
| 19 | dito | 1-hydroxynaphthalene-4-sulfonic acid | scarlet |
| 20 | dito | 1-acetylamino-8-hydroxy-naphthalene-3,6-disulfonic acid | red |
| 21 | dito | 1-acetylamino-8-hydroxy-naphthalene-4,6-disulfonic acid | red |
| 22 | dito | 1-benzoylamino-8-hydroxy-naphthalene-3,6-disulfonic acid | red |
| 23 | dito | 1-benzoylamino-8-hydroxy-naphthalene-4,6-disulfonic acid | red |
| 24 | dito | 2-acetylamino-5-hydroxy-naphthalene-7-sulfonic acid | orange |
| 25 | dito | 3-acetylamino-5-hydroxy-naphthalene-7-sulfonic acid | scarlet |
| 26 | dito | 1-(4'-sulfophenyl)-3-methyl-5-pyrazolone | yellow |
| 27 | dito | 1-(4'-sulfophenyl)-3-carboxy-5-pyrazolone | yellow |

| Bsp. | Naphthylamine Base | Coupler | Shade |
|---|---|---|---|
| 28 | 2-amino-5-(ß-sulfatoethylsulfonyl)-naphthalene-7-sulfonic acid | 1-[4'-(2-sulfatoethylsulfonyl)-phenyl]-3-methyl-5-pyrazolone | yellow |
| 29 | dito | N,N-bis-(2-sulfatoethyl)-3-chloro-aniline | orange |
| 30 | dito | N-(2-sulfatoethyl)-4-methyl-6-hydroxy-pyridone | yellow |
| 31 | dito | 1-hydroxynaphthalene-3,6-disulfonic acid copper complex | blue |
| 32 | dito | 1-hydroxynaphthalene-4-sulfonic acid copper complex | blue |
| 33 | dito | 1-acetylamino-8-naphthol-3,6-disulfonic acid copper complex | blue |
| 34 | dito | N,N-bis-(2-sulfatoethyl)-aniline | orange |
| 35 | dito | 1-hydroxy-3,6-disulfo-8-[5-chloro-3-(3-sulfophenyl)-amino-2,4,6-triazin-1-yl]-amino-naphthalene | red |
| 36 | 2-amino-7-(ß-sulfatoethylsulfonyl)-naphthalene-5-sulfonic acid | 1-acetoacetylamino-3,6-dimethoxy-benzene-4-sulfonic acid | yellow |
| 37 | dito | 1-acetylamino-8-hydroxy-naphthalene 3,6-disulfonic acid | red |
| 38 | dito | 1-acetylamino-8-hydroxy-naphthalene-4,6-disulfonic acid | red |

| Bsp. | Naphthylamine Base | Coupler | Shade |
|---|---|---|---|
| 39 | 2-amino-7-(β-sulfatoethylsulfonyl)-naphthalene-5-sulfonic acid | 1-benzoylamino-8-hydroxy-naphthalene-3,6-disulfonic acid | red |
| 40 | dito | 1-benzoylamino-8-hydroxy-naphthalene-4,6-disulfonic acid | red |
| 41 | dito | 3-acetylamino-5-hydroxy-naphthalene-7-sulfonic acid | scarlet |
| 42 | dito | 1-(4'-sulfophenyl)-3-methyl-5-pyrazolone | yellow |
| 43 | dito | 1-(4'-sulfophenyl)-3-carboxy-5-pyrazolone | yellow |
| 44 | dito | 1-[4'-(2-sulfatoethylsulfonyl)-phenyl]-3-methyl-5-pyrazolone | yellow |
| 45 | dito | N,N-bis-(2-sulfatoethyl)-3-chloro-aniline | orange |
| 46 | dito | N-(2-sulfoethyl)-4-methyl-6-hydroxy-pyridone | yellow |
| 47 | dito | 1-hydroxy-naphthalene-3,6-disulfonic acid copper complex | blue |
| 48 | dito | N,N-bis-(2-sulfatoethyl)-aniline | orange |
| 49 | dito | 1-hydroxy-3,6-disulfo-8-[5-chloro-3-(3-sulfophenyl)-amino-2,4,6-triazin-1-yl]-amino-naphthalene | red |

## Claims

**1.** A process for the preparation of acylamino-naphthalene or of an amino-naphthalene of the general formula (1)

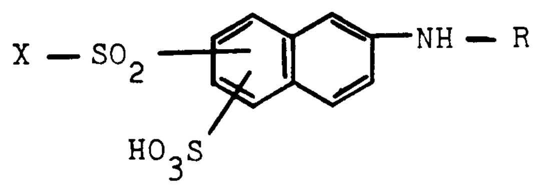

(1)

wherein:

R is a hydrogen atom or a group of the general formula -CO-R' in which
R' is an alkyl, aryl or substituted alkyl or substituted aryl group,
X is a group of the formula $-CH_2-CH_2-Z$ , in which Z is an organic or inorganic substituent capable of being split off by means of an alkaline agent, and

the sulfo group $-SO_3H$ is in the 7-position if the group $X-SO_2-$ is in the 5-position, or the sulfo group is in the 5-position if the group $X-SO_2-$is in the 7-position,
characterized in that a 2-acylamino naphthalene compound of the general formula (2)

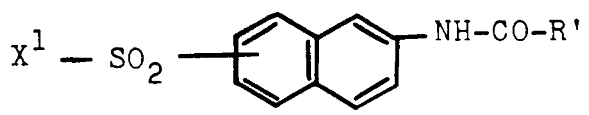

(2)

in which R' is defined as above, $X^1$ has one of the meanings of X or is the β-hydroxyethyl group, and the group $X^1-SO_2-$ is the the 5- or 7-position, is reacted with a sulfonation agent, and then hydrolyzing, if desired to form a compound of formula (1) in which R is hydrogen, the compound of formula (1) in which R' is a group of the formula -CO-R', by diluting with water to a 85 to 100 % sulfuric acid solution and heating it at a temperature between 80 to 120 ° C.

**2.** A process according to claim 1, wherein X is β-chloroethyl, β-sulfatoethyl, β-phosphatoethyl or β-thiosulfatoethyl and $X^1$ is defined as for X or is β-hydroxyethyl.

**3.** A process according to claim 1 or 2, wherein R' is alkyl or 1 to 4 carbon atoms unsubstituted or substituted by phenyl, or is phenyl unsubstituted or substituted by one or more substituents selected from halogen, nitro, alkoxy of 1 to 4 carbon atoms, phenoxy and alkyl of 1 to 4 carbon atoms.

**4.** A process according to one or more of claims 1 to 3, wherein the sulfonation agent is sulfuric acid containing sulfur trioxide (fuming sulfuric acid) at a temperature of between 10 and 50 ° C, preferably 10 to 20 ° C.

**5.** A compound of the general formula (5A)

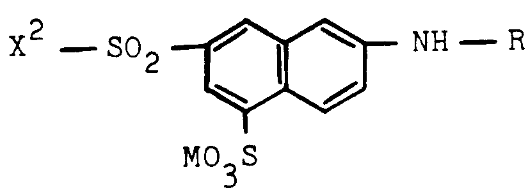

(5A)

wherein:

M is hydrogen or an alkali metal,
R is hydrogen of a group of the general formula -CO-R' in which
R' is an alkyl, aryl or substituted alkyl or substituted aryl group, and
$X^2$ is the vinyl group or a group of the formula $-CH_2-CH_2-Z$, in which Z is an organic or inorganic substituent capable of being split off by means of an alkaline agent.

6. A compound according to claim 5, wherein $X^2$ is $\beta$-chloroethyl, $\beta$-sulfatoethyl, $\beta$-phosphatoethyl or $\beta$-thiosulfatoethyl.

7. A compound according to Claim 5 or 6, wherein R is hydrogen.

8. A compound according to claim 5 or 6, wherein R' is alkyl of 1 to 4 carbon atoms unsubstituted or substituted by phenyl, or is phenyl unsubstituted or substituted by one or more substituents selected from halogen, nitro, alkoxy or 1 to 4 carbon atoms, phenoxy and alkyl of 1 to 4 carbon atoms.

9. A compound according to at least one of claims 5, 7 and 8, wherein $X^2$ is $\beta$-sulfatoethyl.

10. A process for the preparation of a compound of claim 5, characterized in that a 2-acylamino-naphthalene compound of the general formula (2A)

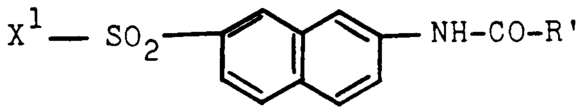
(2A)

in which R' is defined as in claim 5 and $X^1$ has one of the meanings of $X^2$ or is the $\beta$-hydroxyethyl group, is reacted with a sulfonation agent to form a compound of formula (5A) in which R is -CO-R', and then hydrolyzing, if desired to form a compound of formula (5A), in which R is hydrogen, the compound of formula (5A) in which R is -CO-R', by diluting with water to a 85 to 100 % sulfuric acid solution and heating it at a temperature between 80 to 120 ° C, and, if desired to prepare a compound of formula (5A) in which $X^2$ is a vinyl group, reacting the resulting compound of formula (5A) with an alkaline agent.

11. Use of a compound of formula (5A), mentioned and defined in any one of claims 5 to 8, as a starting compound for the preparation of a fibre-reactive dyestuff, in particular when R is hydrogen, as diazo component to prepare an azo dyestuff.

12. A monoazo or disazo dyestuff of the general formula (6a) or (6b)

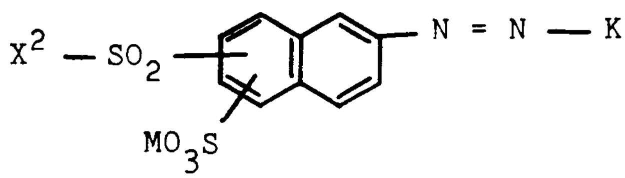
(6a)

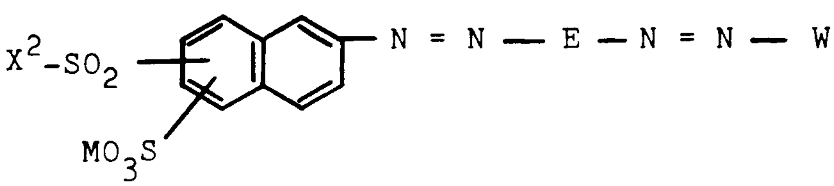
(6b)

wherein:

M is hydrogen or an alkali metal,

$X^2$ is the vinyl group or a group of the formula $-CH_2CH_2-Z$, in which Z is an organic or inorganic substituent capable of being split off by means of an alkaline agent, and

the sulfo group $-SO_3M$ is in the 7-position if the group $X^2-SO_2-$ is in the 5-position, or

the sulfo group is in the 5-position if the group $X^2-SO_2-$isin the 7-position, or

K is the residue of a coupling component, in particular from the benzene, naphthalene, acetoacetic acid arylamine, pyrazolone and pyridone series,

W is the radical K defined above, or is a radical D, D representing a group of the formula (8)

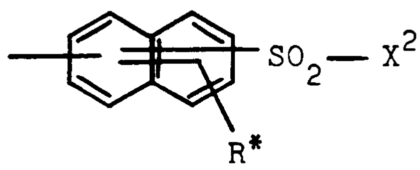

(8)

in which $X^2$ is defined as above and R* is hydrogen or sulfo, or D is phenyl or naphthyl, each unsubstituted or substituted by 1, 2 or 3 substituents independently selected from chloro, fluoro, bromo, sulfo, carboxy, alkoxy of 1 to 4 carbon atoms, alkyl of 1 to 4 carbon atoms, benzoylamino, sulfobenzoylamino, alkanoylamino of 2 to 5 carbon atoms, N-alkylamino of 1 to 4 carbon atoms, N,N-dialkylamino of 1 to 4 carbon atoms in each alkyl and [5-chloro-3-phenylamino-s-triazin-1-yl]-amino, the benzene moiety in the latter group being optionally substituted by sulfo, carboxy, chloro, methyl, ethyl, methoxy, ethoxy and/or nitro and unsubstituted or substituted by one or two groups $-SO_2-X^2$, defined above, and

E is 1,4-phenylene or 1,4-naphthylene optionally substituted with 1 or 2 substituents independently selected from chloro, fluoro, bromo, sulfo, carboxy, alkoxy of 1 to 4 carbon atoms, alkyl of 1 to 4 carbon atoms, benzoylamino, sulfobenzoylamino, alkanoylamino of 2 to 5 carbon atoms, ureido and N,N-dialkylamino of 1 to 4 carbon atoms in each alkyl, or is 2,6- or 2,7-naphthylene substituted in the 1-position by hydroxy and substituted by one or two sulfo groups, or

E represents a 1-hydroxy-8-amino-3,6- or -4,6-disulfonaphth-2,7-ylene;

or a metal-complex dyestuff thereof.

**13.** An azo compound according to claim 12, wherein $X^2$ is $\beta$-chloroethyl, $\beta$-sulfatoethyl, $\beta$-phosphatoethyl or $\beta$-thiosulfatoethyl.

**14.** An azo compound according to claim 12 or 13, wherein $X^2$ is $\beta$-sulfatoethyl.

**15.** A monoazo compound according to at least one of claims 12 to 14, of the general formula (6a) wherein K is a group of the general formula (7a)

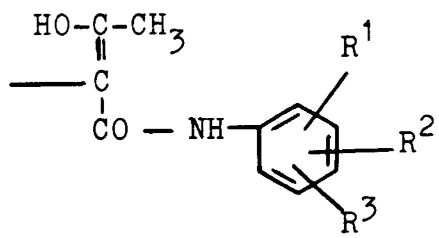

(7a)

wherein $R^1$, $R^2$ and $R^3$ have meanings which may be identical to or different from one another, and $R^1$ is hydrogen, hydroxy or a group $-SO_2-X^2$ wherein $X^2$ has one of the above meanings, $R^2$ is hydrogen, chloro, bromo, alkyl of 1 to 4 carbon atoms, alkoxy of 1 to 4 carbon atoms or sulfo, and $R^3$ is hydrogen, chloro, alkyl of 1 to 4 carbon atoms, alkoxy of 1 to 4 carbon atoms or sulfo, or a metal complex thereof.

**16.** A monoazo compound according to at least one of claims 12 to 14, of the general formula (6a) wherein K is a group of the general formula (7b)

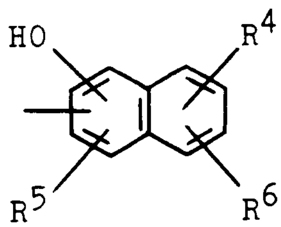

(7b)

wherein the free bond is in the ortho-position relative to the hydroxy group, $R^4$ is hydrogen, alkyl of 1 to 4 carbon atoms, sulfo, amino, hydroxy, benzoylamino, sulfobenzoylamino, alkanoylamino of 2 to 5 carbon atoms, N-alkylamino of 1 to 4 carbon atoms, 3-phenylamino-5-chloro-s-triazin-1-ylamino, the benzene moiety in the latter group being optionally substituted by sulfo, carboxy, chloro, methyl, ethyl, methoxy, ethoxy and/or nitro, $R^5$ is hydrogen or sulfo and $R^6$ is hydrogen or sulfo, where $R^4$, $R^5$ and $R^6$ may have meanings which are identical to or different from another, or a metal complex thereof.

**17.** A monoazo compound according to at least one of claims 12 to 14, of the general formula (6a) wherein K is a group of the general formula (7c)

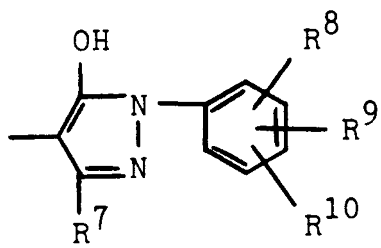

(7c)

in which $R^7$ is alkyl of 1 to 4 carbon atoms, alkoxy of 1 to 4 carbon atoms, carboxy or carbalkoxy of 2 to 5 carbon atoms, $R^8$ is hydrogen, sulfo, alkyl of 1 to 4 carbon atoms, alkoxy of 1 to 4 carbon atoms, chloro, bromo, hydroxy or $-SO_2-X^2$ , $R^9$ is hydrogen, sulfo, alkyl of 1 to 4 carbon atoms, alkoxy of 1 to 4 carbon atoms or chloro, and $R^{10}$ is hydrogen or sulfo, or a metal complex thereof.

**18.** A monoazo compound according to at least one of claims 12 to 14, of the general formula (6a) wherein K is a group of the general formula (7d)

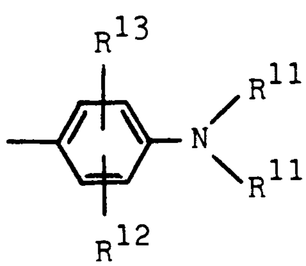

(7d)

wherein each $R^{11}$ is independently selected from hydrogen and alkyl of 1 to 4 carbon atoms, unsubstituted or substituted by sulfo, carboxy, hydroxy, sulfato or alkoxy of 1 to 4 carbon atoms, $R^{12}$ is hydroxy, chloro, bromo, alkyl of 1 to 4 carbon atoms, alkoxy of 1 to 4 carbon atoms, sulfo, N-alkylamino of 1 to 4 carbons, alkanoylamino of 2 to 5 carbon atoms, ureido and $-SO_2-X^2$, and $R^{13}$ is hydrogen chloro, alkyl of 1 to 4 carbon atoms, alkoxy of 1 to 4 carbon atoms and sulfo.

**19.** A monoazo dyestuff according to at least one of claims 12 to 14, wherein K is a group of the formula (7e) or (7f)

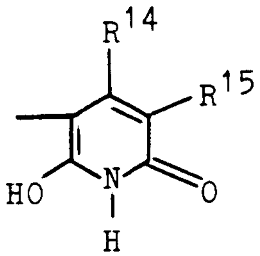

(7e)

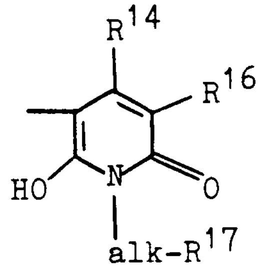

(7f)

in which

- $R^{14}$ is alkyl of 1 to 4 carbon atoms, such as methyl, phenyl or hydrogen, preferably hydrogen or carbamoyl.
- $R^{15}$ is hydrogen, chloro, bromo, sulfo, cyano, carbamoyl, carboxy or sulfamoyl, preferably hydrogen or carbamoyl,
- $R^{16}$ is hydrogen, chloro, bromo, sulfo, cyano, carbamoyl, carboxy or sulfamoyl, preferably hydrogen or carbamoyl,

and

- $R^{17}$ is hydroxy, sulfo, sulfato, amino, alkylamino of 1 to 4 carbon atoms, alkoxy of 1 to 4 carbon atoms and chloro, preferably methoxy or sulfo.

**20.** A monoazo dyestuff according to at least one of claims 12 to 14, wherein K is a group of the general formula (7g)

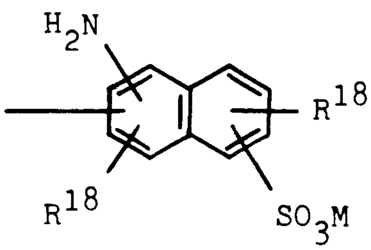

(7g)

in which the free bond is in the ortho- or para-position relatively to the amino group, and each $R^{18}$ is independently selected from hydrogen and sulfo, and M is defined in claim 12.

**21.** A 1:1-copper-, 1:2-cobalt- or 1:2-chromium complex monoazo dyestuff, according to at least one of claims 12 to 14, of the compound of the general formula (6A)

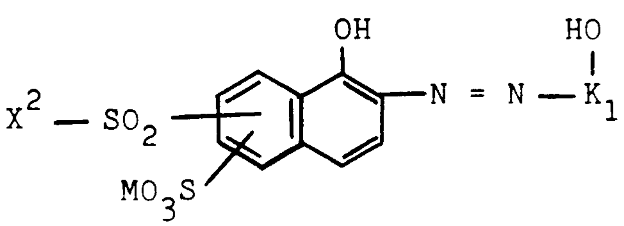

(6A)

in which M and $X^2$ and the positions of the groups $X^2$-$SO_2$- and $MO_3S$- are defined as in claim 12 for formula (6a) and $K_1$ is an unsubstituted or substituted moiety of a hydroxy-coupling component selected from the series mentioned in claim 12 for the radical K, and the hydroxy group of $K_1$ is in the ortho-, respectively vicinal, position to the azo group.

**22.** A 1:1-copper complex monoazo dyestuff according to claim 21, wherein the radical -$K_1$-OH is a group of the general formula (7a), (7b) or (7c) shown in defined in claims 15 to 17.

**23.** A copper complex monoazo dyestuff according to claim 21, of the general formula (6B)

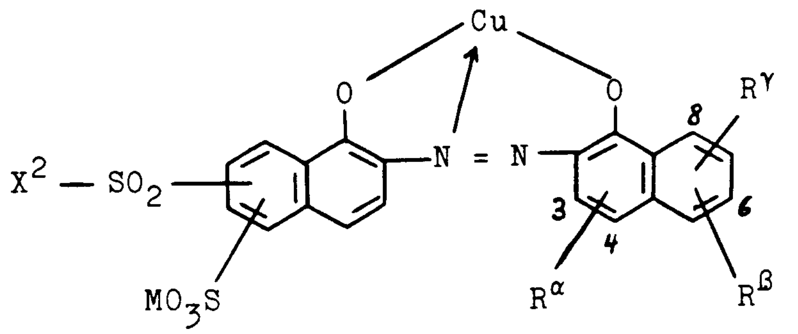

(6B)

wherein the group $X^2$-$SO_2$- is in the 5- or 7-position and the group $MO_3S$- is in the 7- or 5-position, respectively,
$R^\alpha$ is sulfo in the 4-position and $R^\beta$ and $R^\gamma$ are both hydrogen, or
$R^\alpha$ is sulfo in the 3-position, $R^\beta$ is sulfo in the 6-position and $R^\gamma$ is hydrogen, or
$R^\alpha$ is sulfo in the 3-position, $R^\beta$ is sulfo in the 6-position and $R^\gamma$ is acetylamino in the 8-position.

**24.** A process for the preparation of a monoazo compound of formula (6a) mentioned and defined in claim 12, or of a disazo compound of formula (6b) mentioned and defined in claim 12, the formula member W, however, having the meaning of K, and the formula member E, however, representing the optionally substituted 1,4-phenylene or 1,4-naphthylene, or the 1-hydroxy-mono- or -disulfo-2,6-or -2,7-naphthylene, as defined in claim 12, characterized in that an aminonaphthalene of the general formula (5)

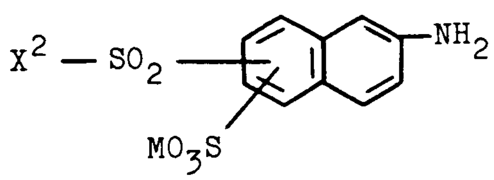

(5)

wherein M and $X^2$ are defined as in claim 12 and the substituent $X^2$-$SO_2$- is in the 5-position if the group $MO_3S$- is in the 7-position, or the substituent $X^2$-$SO_2$-is in the 7-position if the group $MO_3S$- is in the 5-position, or a compound of the general formula (9)

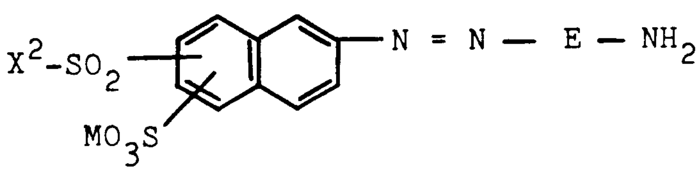

(9)

in which $X^2$, M and E are defined as above, is diazotized and coupled with a compound of the general formula H-K in which K is defined as in any one of claims 12 to 20.

**25.** A process for the preparation of a disazo compound of the general formula (6b), mentioned and defined as in claim 12, the formula member W, however, having the meaning of D, and the formula member E, however, representing the 1-hydroxy-8-amino-3,6-disulfo-naphth-2,7-ylene or 1-hydroxy-8-amino-4,6-disulfo-2,7-naphthylene group, characterized in that a diazonium salt of an amino compound of formula (5)

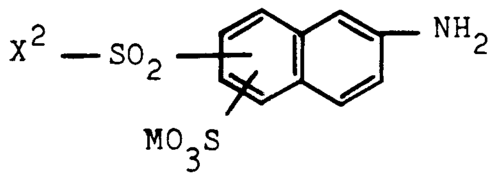

(5)

wherein M and $X^2$ are defined as in claim 12 and the substituent $X^2$-$SO_2$- is in the 5-position if the group $MO_3S$- is in the 7-position, or the substituent $X^2$-$SO_2$-is in the 7-position if the group $MO_3S$- is in the 5-position, and a diazonium salt of an amino compound of the general formula $H_2N$-D in which D has one of the meanings given in claim 12, are coupled, in a desired order, with a coupling compound of the general formula H-E-H in which E is 1-hydroxy-8-amino-3,6- or -4,6-disulfo-naphth-2,7-ylene, while the coupling reaction is first carried out in a strongly acidic pH-range and then in a weakly acidic to very weakly alkaline pH-range.

**26.** A process for the preparation of a metal complex, preferably 1:1-copper complex, or a monoazo or disazo compound of at least one of claims 12 to 23, characterized in that a metal-free monoazo or disazo dyestuff of at least one of claims 12 to 19 is reacted with a metal donor agent, preferably a copper yielding agent in the presence of an oxidizing agent.

**27.** Use of an azo compound of at least one of claims 12 to 23 as a dye for dyeing or printing hydroxy and/or carboxamido-containing fiber material.

**28.** A process for dyeing or printing a hydroxy and/or carboxamido-containing fiber material by applying a dye to the material and fixing it by means of heat and/or by means of an acid-binding agent, characterized in that the dye is used as an azo compound of at least one of claims 12 to 23.

**Patentansprüche**

**1.** Verfahren zur Herstellung eines Acylaminonaphthalins oder eines Aminonaphthalins der allgemeinen Formel (1)

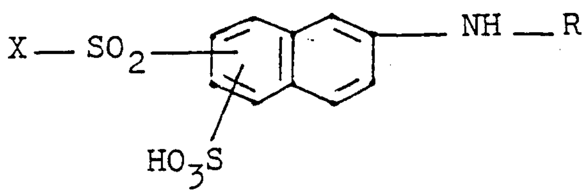

(1)

in welcher R Wasserstoff oder ein Rest der allgemeinen Formel -CO-R', worin R' einen Alkyl-, Aryl-, substituierten Alkyl- oder substituierten Arylrest bedeutet,und X einen Rest der Formel $-CH_2-CH_2-Z$, worin Z ein organischer oder anorganischer Substituent ist, der mittels eines alkalischen Agens abgespalten werden kann, bedeutet und die Sulfogruppe $-SO_3H$ sich in 7-Stellung befindet, wenn die Gruppe X-$SO_2$- in 5-Stellung ist, oder die Sulfogruppe sich in 5-Stellung befindet, wenn die Gruppe X-$SO_2$- in 7-Stellung ist, dadurch gekennzeichnet, daß eine 2-Acylaminonaphthalinverbindung der allgemeinen Formel (2)

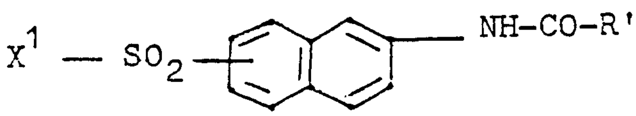

(2)

in welcher R' wie vorher definiert ist, $X^1$ eine der Bedeutungen von X hat oder die ß-Hydroxyäthylgruppe ist und die Gruppe $X^1$-$SO_2$-sich in 5- oder 7-Stellung befindet, mit einem Sulfonierungsagens zur Reaktion gebracht wird und dann, wenn es erwünscht ist ,eine Verbindung der Formel (1) zu bilden, in welcher R Wasserstoff ist, die Verbindung der Formel (1), in welcher R' ein Rest der Formel -CO-R' ist,

durch Verdünnen mit Wasser auf eine 85 bis 100 %ige Schwefelsäurelösung und Erhitzen auf eine Temperatur zwischen 80 und 120°C hydrolysiert wird.

2. Verfahren nach Anspruch 1, wobei X $\beta$-Chloräthyl, $\beta$-Sulfatoäthyl, $\beta$-Phosphatoäthyl oder $\beta$-Thiosulfatoäthyl ist und $X^1$ und X definiert ist oder $\beta$-Hydroxyäthyl bedeutet.

3. Verfahren nach Anspruch 1 oder 2, wobei R' unsubstituiertes oder durch Phenyl substituiertes Alkyl mit 1 bis 4 C-Atomen oder unsubstituiertes oder durch einen oder mehrere Substituenten, ausgewählt aus Halogen, Nitro, Alkoxy mit 1 bis 4 C-Atomen, Phenoxy und Alkyl mit 1 bis 4 C-Atomen, substituiertes Phenyl ist.

4. Verfahren nach einem oder mehreren der Ansprüche 1 bis 3, wobei das Sulfonierungsagens Schwefeltrioxyd enthaltende Schwefelsäure (rauchende Schwefelsäure) ist, bei einer Temperatur zwischen 10 und 50 ° C vorzugsweise zwischen 10 bis 20 ° C.

5. Eine Verbindung der allgemeinen Formel (5A)

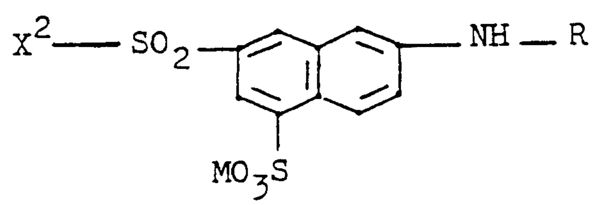

(5A)

in welcher M Wasserstoff oder ein Alkalimetall, R Wasserstoff oder ein Rest der allgemeinen Formel -CO-R', worin R' ein Alkyl-, Aryl-, substituierter Alkyl- oder substituierter Arylrest ist,und $X^2$ den Vinylrest oder einen Rest der Formel $-CH_2-CH_2-Z$, worin Z ein organischer oder anorganischer Substituent ist, der mittels eines alkalischen Agens abgespalten werden kann, bedeutet.

6. Eine Verbindung nach Anspruch 5, in welcher $X^2$ $\beta$-Chloräthyl, $\beta$-Sulfatoäthyl, $\beta$-Phosphatoäthyl oder $\beta$-Thiosulfatoäthyl ist.

7. Eine Verbindung nach Anspruch 5 oder 6, in welcher R Wasserstoff ist.

8. Eine Verbindung nach Anspruch 5 oder 6, in welcher R' ein unsubstituiertes oder durch Phenyl substituiertes Alkyl mit 1 bis 4 C-Atomen oder ein unsubstituiertes oder durch einen oder mehrere Substituenten, ausgewählt aus Halogen` Nitro, Alkoxy mit 1 bis 4 C-Atomen, Phenoxy und Alkyl mit 1 bis 4 C-Atomen, substituiertes Phenyl bedeutet.

9. Eine Verbindung nach mindestens einem der Ansprüche 5, 7 und 8, in welcher $X^2$ $\beta$-Sulfatoäthyl ist.

10. Verfahren zur Herstellung einer Verbindung nach Anspruch 5, dadurch gekennzeichnet, dass eine 2-Acylaminonaphthalinverbindung der allgemeinen Formel (2A)

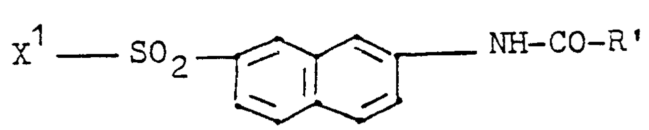

(2A)

in welcher R' wie in Anspruch 5 definiert ist und $X^1$ eine der Bedeutungen von $X^2$ hat oder die $\beta$-Hydroxyäthylgruppe ist, mit einem Sulfonierungsagens zur Reaktion gebracht wird, um eine Verbindung der Formel (5A), in welcher R -CO-R' ist, zu bilden,und dann, wenn es erwünscht ist, eine Verbindung der Formel (5A), in welcher R Wasserstoff ist, zu bilden, die Verbindung der Formel (5A), in welcher R -CO-R' ist, durch Verdünnen mit Wasser auf eine 85 bis 100 %ige Schwefelsäurelösung und Erhitzen auf eine Temperatur zwischen 80 und 120°C hydrolysiert wird, und, falls es erwünscht ist, eine Verbindung der Formel (5A), in welcher $X^2$ eine Vinylgruppe ist, herzustellen, die resultierende

Verbindung der Formel (5A) mit einem alkalischen Agens zur Reaktion gebracht wird.

**11.** Verwendung einer Verbindung der Formel (5A), wie sie in irgendeinem der Ansprüche 5 bis 8 beschrieben und definiert ist, als Ausgangsverbindung für die Herstellung eines faserreaktiven Farbstoffes, insbesondere im Fall, dass R Wasserstoff ist, als Diazokomponente, um einen Azofarbstoff herzustellen.

**12.** Ein Monoazo- oder Disazofarbstoff der allgemeinen Formel (6a) oder (6b)

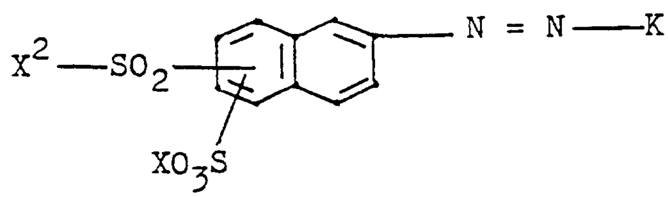

(6a)

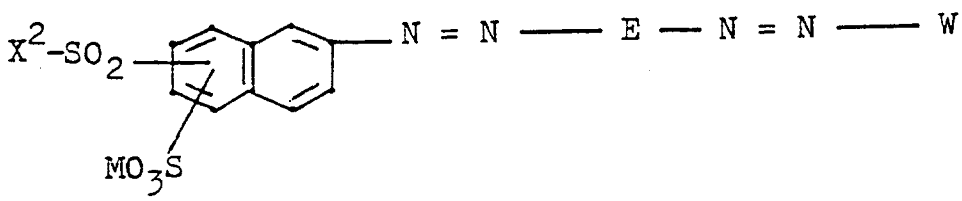

(6b)

in welcher M Wasserstoff oder ein Alkalimetall und $X^2$ die Vinylgruppe oder ein Rest der Formel $-CH_2-CH_2-Z$, in welcher Z ein organischer oder anorganischer Substituent, der mittels eines alkalischen Agens abgespalten werden kann, ist, bedeutet,und die Sulfogruppe $-SO_3M$ sich in 7-Stellung befindet, wenn die Gruppe $X^2-SO_2$-in 5-Stellung ist,oder die Sulfogruppe sich in 5-Stellung befindet, wenn die Gruppe $X^2-SO_2$- in 7-Stellung ist, oder K der Rest einer Kupplungskomponente, insbesondere aus der Benzol-, Naphthalin-, Acetessigsäurearylamin-, Pyrazolon- und Pyridon-Reihe, ist, W der Rest K ist, wie vorher definiert ,oder der Rest D ist, wobei D eine Gruppe der Formel (8)

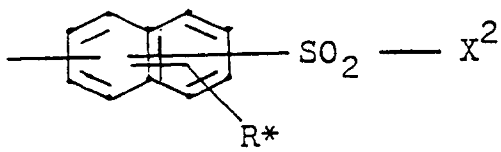

(8)

bedeutet, in welcher $X^2$ wie vorher definiert ist und R* Wasserstoff oder die Sulfogruppe ist, oder D Phenyl oder Naphthyl ist, von denen jedes unsubstituiert oder durch 1, 2 oder 3 Substituenten substituiert sein kann, welche unabhängig voneinander ausgewählt sind aus Chlor, Fluor, Brom,Sulfo, Carboxy,
Alkoxy mit 1 bis 4 C-Atomen, Alkyl mit 1 bis 4 C-Atomen, Benzoylamino, Sulfobenzoylamino, Alkanoylamino mit 2 bis 5 C-Atomen, N-Alkylamino mit 1 bis 4 C-Atomen, N,N-Dialkylamino mit 1 bis 4 C-Atomen in jedem Alkyl und [5-Chlor-3-phenylamino-2-triazin-1-yl]amino, wobei der Benzolrest in der letzteren Gruppe gegebenenfalls durch Sulfo, Carboxy, Chlor, Methyl, Äthyl, Methoxy,
Äthoxy und/oder Nitro substituiert und unsubstituiert oder durch eine oder zwei $-SO_2-X^2$ -Gruppen, wie oben definiert, substituiert sein kann ,und E 1,4-Phenylen oder 1,4-Naphthylen ist, das gegebenenfalls durch 1 oder 2 Substituenten unabhängig voneinander ausgewählt aus Chlor, Brom, Fluor, Sulfo, Carboxy,
Alkoxy mit 1 bis 4 C-Atomen, Alkyl mit 1 bis 4 C-Atomen, Benzoylamino, Sulfobenzoylamino, Alkanoylamino mit 2 bis 5 C-Atomen, Ureido und N,N-Dialkylamino mit 1 bis 4 C-Atomen in jedem Alkyl substituiert ist, oder 2,6- oder 2,7-Naphthylen, substituiert in 1-Stellung durch Hydroxy und substituiert durch eine oder zwei Sulfogruppen, ist, oder E ein 1-Hydroxy-8-amino-3,6 - oder -4,6-disulfonaphth-2,7-ylen bedeutet ,oder ein Metallkomplexfarbstoff davon.

13. Eine Azoverbindung nach Anspruch 12, in welcher $X^2$ β-Chloräthyl, β-Sulfatoäthyl, β-Phosphatoäthyl oder β-Thiosulfatoäthyl ist.

14. Eine Azoverbindung nach Anspruch 12 oder 13, in welcher $X^2$ β-Sulfatoäthyl ist.

15. Eine Monoazoverbindung nach mindestens einem der Ansprüche 12 bis 14 der allgemeinen Formel (6a), in welcher K ein Rest der allgemeinen Formel (7a)

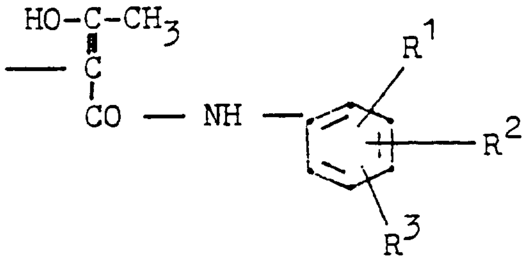

(7a)

ist, in welcher $R^1$, $R^2$ und $R^3$ gleiche oder voneinander verschiedene Bedeutungen haben können und $R^1$ Wasserstoff, Hydroxy oder eine Gruppe $-SO_2-X^2$, worin $X^2$ eine der oben angegebenen Bedeutungen hat, $R^2$ Wasserstoff, Chlor, Brom, Alkyl mit 1 bis 4 C-Atomen, Alkoxy mit 1 bis 4 C-Atomen oder Sulfo und $R^3$ Wasserstoff, Chlor, Alkyl mit 1 bis 4 C-Atomen, Alkoxy mit 1 bis 4 C-Atomen oder Sulfo bedeutet, oder ein Metallkomplex davon.

16. Eine Monoazoverbindung nach mindestens einem der Ansprüche 12 bis 14 der allgemeinen Formel (6a), in welcher K ein Rest der allgemeinen Formel (7b)

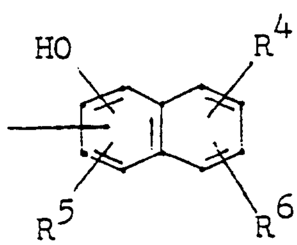

(7b)

ist, in welcher sich die freie Bindung in o-Stellung in bezug auf die. Hydroxygruppe befindet, $R^4$ Wlasserstoff, Alkyl mit 1 bis 4 C-Atomen, Sulfo, Amino, Hydroxy, Benzoylamino, Sulfobenzoylamino, Alkanoylamino mit 2 bis 5 C-Atomen, N-Alkylamino mit 1 bis 4 C-Atomen oder 3-Phenylamino-5-chlor-s-triazin-1-ylamino bedeutet, wobei der Benzolrest in der letzteren Gruppe gegebenenfalls durch Sulfo, Carboxy, Chlor, Methyl, Äthyl, Methoxy, Äthoxy und/oder Nitro substituiert sein kann, $R^5$ Wasserstoff oder Sulfo und $R^6$ Wasserstoff oder Sulfo bedeutet, wobei $R^4$, $R^5$ und $R^6$ Bedeutungen haben können, die identisch zueinander oder voneinander verschieden sind, oder ein Metallkomplex davon.

17. Eine Monoazoverbindung nach mindestens einem der Ansprüche 12 bis 14 der allgemeinen Formel (6a), in welcher K ein Rest der allgemeinen Formel (7c)

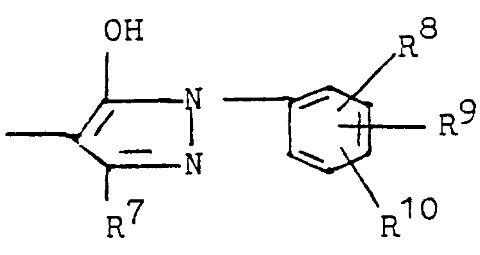

(7c)

ist,in welcher $R^7$ Alkyl mit 1 bis 4 C-Atomen,Alkoxy mit 1 bis 4 C-Atomen, Carboxy oder Carbalkoxy mit

2 bis 5 C-Atomen, $R^8$ Wasserstoff, Sulfo, Alkyl mit 1 bis 4 C-Atomen, Alkoxy mit 1 bis 4 C-Atomen, Chlor, Brom, Hydroxy oder $-SO_2-X^2$, $R^9$ Wasserstoff, Sulfo, Alkyl mit 1 bis 4 C-Atomen, Alkoxy mit 1 bis 4 C-Atomen oder Chlor und $R^{10}$ Wasserstoff oder Sulfo bedeutet,oder ein Metallkomplex davon.

**18.** Eine Monoazoverbindung nach mindestens einem der Ansprüche 12 bis 14 der allgemeinen Formel (6a), in welcher K ein Rest der allgemeinen Formel (7d)

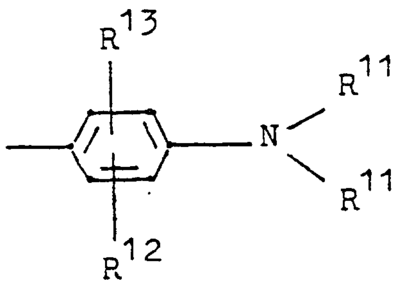

(7d)

ist, in welcher jedes $R^{11}$ unabhängig ausgewählt ist aus Wasserstoff und Alkyl mit 1 bis 4 C-Atomen, des unsubstituiert oder substituiert ist durch Sulfo, Carboxy, Hydroxy, Sulfato oder Alkoxy mit 1 bis 4 C-Atomen, $R^{12}$ Hydroxy, Chlor, Brom, Alkyl mit 1 bis 4 C-Atomen, Alkoxy mit 1 bis 4 C-Atomen, Sulfo, N-Alkylamino mit 1 bis 4 C-Atomen, Alkanoylamino mit 2 bis 5 C-Atomen, Ureido und $-SO_2-X^2$ und $R^{13}$ Wasserstoff, Chlor, Alkyl mit 1 bis 4 C-Atomen, Alkoxy mit 1 bis 4 C-Atomen und die Sulfogruppe bedeutet.

**19.** Ein Monoazofarbstoff nach mindestens einem der Ansprüche 12 bis 14, wobei K ein Rest der allgemeinen Formel (7e) oder (7f)

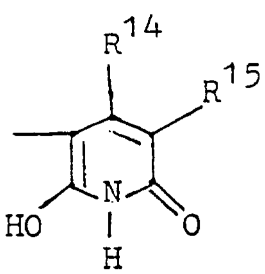

(7e)

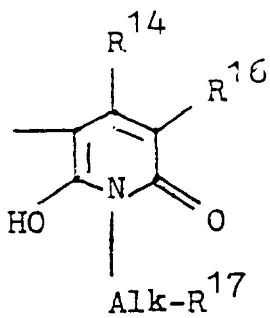

(7f)

ist, in welcher $R^{14}$ Alkyl mit 1 bis 4 C-Atomen, wie Methyl, Phenyl oder Wasserstoff, vorzugsweise Wasserstoff oder Carbamoyl, $R^{15}$ Wasserstoff, Chlor, Brom, Sulfo, Cyano, Carbamoyl, Carboxy oder Sulfamoyl, vorzugsweise Wasserstoff oder Carbamoyl, $R^{16}$ Wasserstoff, Chlor, Brom, Sulfo, Cyano, Carbamoyl, Carboxy oder Sulfamoyl, vorzugsweise Wasserstoff oder Carbamoyl, und $R^{17}$ Hydroxy, Sulfo, Sulfato, Amino, Alkylamino mit 1 bis 4 C-Atomen, Alkoxy mit 1 bis 4 C-Atomen und Chlor, vorzugsweise Methoxy oder Sulfo, bedeutet.

**20.** Ein Monoazofarbstoff nach mindestens einem der Ansprüche 12 bis 14, wobei K ein Rest der allgemeinen Formel (7g)

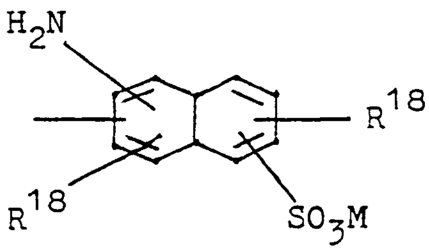

(7g)

ist, in welcher sich die freie Bindung in o- oder p-Stellung in bezug auf die Aminogruppe befindet und jedes $R^{18}$ unabhängig ausgewählt ist aus Wasserstoff und Sulfo und M wie in Anspruch 12 definiert ist.

**21.** Ein 1 : 1-Kupfer-, 1 : 2-Cobalt- oder 1 : 2-Chromkomplex-Monoazofarbstoff nach mindestens einem der Ansprüche 12 bis 14 der Verbindung der allgemeinen Formel (6A)

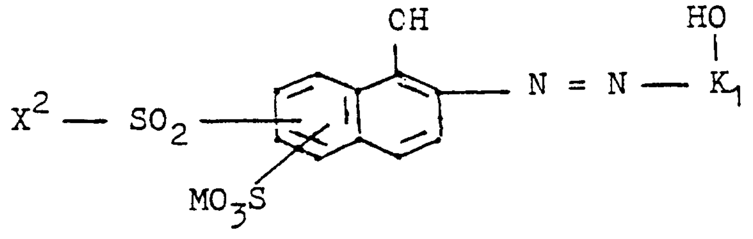

(6A)

in welcher M und $X^2$ und die Stellungen der Gruppen $X^2$-$SO_2$-und $MO_3S$- wie in Anspruch 12 für Formel (6a) definiert sind und $K_1$ ein unsubstituierter oder substituierter Rest einer Hydroxykupplungskomponente,ausgewählt aus den in Anspruch 12 für den Rest K angegebenen Reihe ist und die Hydroxygruppe von $K_1$ sich in o-Stellung bzw. Nachbarstellung zu der Stellung der Azogruppe befindet.

**22.** Ein 1 : 1-Kupferkomplex-Monoazofarbstoff nach Anspruch 21, wobei der Rest -$K_1$-OH einen Rest der allgemeinen Formel (7a), (7b) oder (7c),wie in den Ansprüchen 15 bis 17 angegeben und definiert,ist, bedeutet.

**23.** Ein Kupferkomplex-Monoazofarbstoff nach Anspruch 21 der allgemeinen Formel (6B)

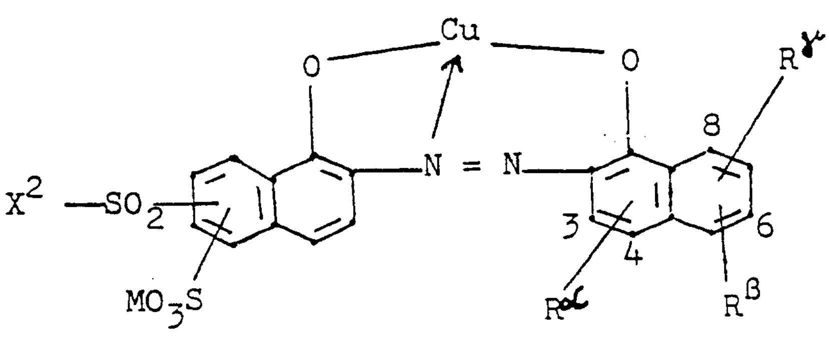

(6B)

in welcher sich die Gruppe $X^2$-$SO_2$- in 5- oder 7-Stellung befindet bzw. sich die Gruppe $MO_3S$- in 7- oder 5-Stellung befindet, $R^\alpha$ die Sulfogruppe in 4-Stellung und $R^\beta$ und $R^\gamma$ beide Wasserstoff oder $R^\alpha$ die Sulfogruppe in 3-Stellung, $R^\beta$ die Sulfogruppe in 6-Stellung und $R^\gamma$ Wasserstoff oder $R^\alpha$ die Sulfogruppe in 3-Stellung $R^\beta$ die Sulfogruppe in 6-Stellung und $R^\gamma$ Acetylamino in 8-Stellung ist.

**24.** Verfahren zur Herstellung einer Monoazoverbindung der Formel (6a), wie in Anspruch 12 beschrieben und definiert, oder einer Disazoverbindung der Formel (6b), wie in Anspruch 12 beschrieben und definiert, wobei der Formelbestandteil W jedoch die Bedeutung von K und der Formelbestandteil E jedoch ein gegebenenfalls substituiertes 1,4-Phenylen oder 1,4-Naphthylen oder das 1-Hydroxymono-

oder -disulfo-2,6- oder -2,7-naphthylen, wie in Anspruch 12 definiert, bedeutet, dadurch gekennzeichnet, daß ein Aminonaphthalin der allgemeinen Formel (5)

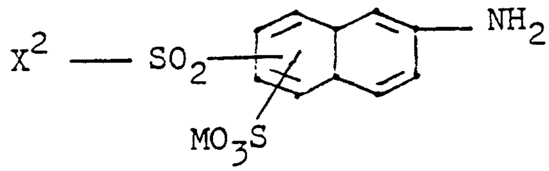
(5)

in welcher M und $X^2$ wie in Anspruch 12 definiert sind,und der Substituent $X^2$-$SO_2$- sich in 5-Stellung befindet, wenn die Gruppe $MO_3S$- in 7-Stellung ist,oder der Substituent $X^2$-$SO_2$- sich in 7-Stellung befindet, wenn die Gruppe $MO_3S$- in 5-Stellung ist,oder eine Verbindung der allgemeinen Formel (9)

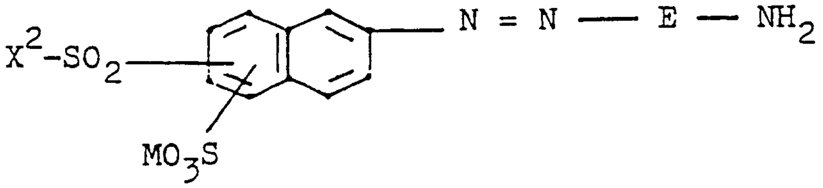
(9)

in welcher $X^2$, M und E wie vorher definiert sind, diazotiert und mit einer Verbindung der allgemeinen Formel H-K, in welcher K wie in irgendeinem der Ansprüche 12 bis 20 definiert ist, gekuppelt wird.

**25.** Verfahren zur Herstellung einer Disazoverbingung allgemeinen Formel (6b), wie in Anspruch 12 angegeben und definiert, wobei der Formelbestandteil W jedoch die Bedeutung von D und der Formelbestandteil E jedoch die Gruppe 1-Hydroxy-8-amino-3,6-disulfonaphth-2,7-ylen oder 1-Hydroxy-8-amino-4,6-disulfo2,7-naphthylen bedeutet, dadurch gekennzeichnet, daß das Diazoniumsalz einer Aminoverbindung der Formel (5)

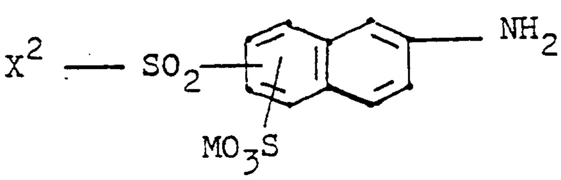
(5)

in welcher M und $X^2$ wie in Anspruch 12 definiert sind und der Substituent $X^2$-$SO_2$- sich in 5-Stellung befindet, wenn sich die Gruppe $MO_3S$- in 7-Stellung befindet oder der Substituent $X^2$-$SO_2$-sich in 7-Stellung befindet, wenn sich die Gruppe $MO_3S$- in 5-Stellung befindet,und ein Diazoniumsalz einer Aminoverbingdung der allgemeinen Formel $H_2N$-D, in welcher D eine der in Anspruch 12 angegebenen Bedeutungen hat, in jeder gewünschten Weise mit einer Kupplungskomponente der allgemeinen Formel H-E-H, in welcher E 1-Hydroxy-8-amino-3,6- oder -4,6-disulfonaphth-2,7-ylen ist, gekuppelt wird, wobei die Kupplungsreaktion zuerst in einem stark sauren pH-Bereich und dann in einem schwach sauren bis sehr schwach alkalischen pH-Bereich ausgeführt wird.

**26.** Verfahren zur Herstellung eines Metallkomplexes, vorzugsweise eines 1 : 1-Kupferkomplexes, einer Monoazo- oder Disazoverbindung nach mindestens einem der Ansprüche 12 bis 23, dadurch gekennzeichnet, daß ein metallfreier Monoazo- oder Disazoverbindung nach mindestens einem der Ansprüche 12 bis 19 mit einem Metall-Donor-Agens, vorzugsweise mit einem Kupfer abgebenden Agens, in Gegenwart eines Oxydationsmittels zur Reaktion gebracht wird.

**27.** Verwendung einer Azoverbindung nach mindestens einem der Ansprüche 12 bis 23 als Farbstoff zum Einfärben oder Bedrucken eines Hydroxy und/oder Carboxamido enthaltenden Fasermaterials.

28. Verfahren zum Einfärben oder Bedrucken eines Hydroxy und/oder Carboxamido enthaltenden Fasermaterials durch Auftragen eines Farbstoffes auf das Material und Fixieren desselben mittels Hitze und/oder mittels eines säurebindenden Agens, dadurch gekennzeichnet, daß der verwendete Farbstoff eine Azoverbindung nach mindestens einem der Ansprüche 12 bis 23 ist.

## Revendications

1. Procédé pour préparer un acylaminonaphtalène ou un aminonaphtalène de formule générale (1)

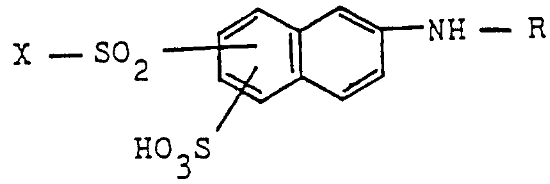

(1)

dans laquelle :

R est un atome d'hydrogène ou un groupe de formule générale -CO-R', dans laquelle R' est un groupe alkyle, aryle ou alkyle substitué ou aryle substitué,

X est un groupe de formule $-CH_2-CH_2-Z$ dans laquelle Z est un substituant organique ou minéral éliminable à l'aide d'un agent alcalin, et

le groupe sulfo $-SO_3H$ est en position 7 si le groupe $X-SO_2-$ est en position 5, ou bien

le groupe sulfo est en position 5 si le groupe $X-SO_2-$ est en position 7,

caractérisé en ce qu'on fait réagir un composé acylamino-2 naphtalène de formule générale (2)

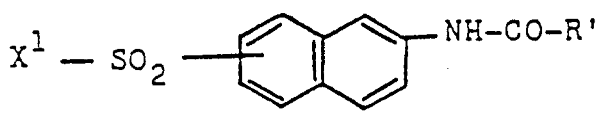

(2)

dans laquelle R' est tel que défini ci-dessus, $X^1$ a l'une des significations de X ou est de préférence le groupe $\beta$-hydroxyéthyle, et le groupe $X^1-SO_2-$ est en position 5 ou 7,
avec un agent de sulfonation, puis on procède à une hydrolyse, si on souhaite former un composé de formule (1) dans laquelle R est un hydrogène, du composé de formule (1) dans laquelle R' est un groupe de formule -CO-R', en diluant à l'eau jusqu'à obtenir une solution d'acide sulfurique à 85 à 100 %, puis chauffage à une température de 80 à 120 ° C.

2. Procédé selon la revendication 1, dans lequel X est le radical $\beta$-chloroéthyle, $\beta$-sulfatoéthyle, $\beta$-phosphatoéthyle ou $\beta$-thiosulfatoéthyle, et $X^1$ est tel que défini pour X, ou encore est le radical $\beta$-hydroxyéthyle.

3. Procédé selon la revendication 1 ou 2, dans lequel R' est un radical alkyle ayant de 1 à 4 atomes de carbone, éventuellement substitué par des radicaux phényle, ou encore est un radical phényle éventuellement substitué par un ou plusieurs substituants choisis parmi les halogènes et les radicaux nitro, alcoxy ayant de 1 à 4 atomes de carbone, phénoxy et alkyle ayant de 1 à 4 atomes de carbone.

4. Procédé selon l'une ou plusieurs des revendications 1 à 3, dans lequel l'agent de sulfonation est l'acide sulfurique contenant du trioxyde de soufre (acide sulfurique fumant) ,à une température comprise entre 10 et 50 ° C et de préférence entre 10 et 20 ° C.

**5.** Composé de formule générale (5A)

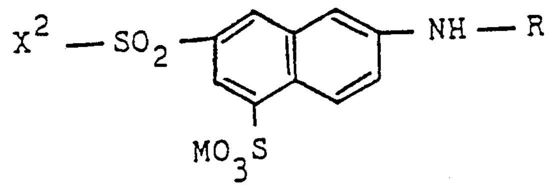

(5A)

dans laquelle :

M est un hydrogène ou un métal alcalin,

R est un hydrogène ou un groupe de formule générale -CO-R', dans laquelle R' est un radical alkyle, aryle, ou alkyle substitué ou aryle substitué, et

$X^2$ est un groupe vinyle ou encore un groupe de formule $-CH_2-CH_2-Z$ dans laquelle Z est un substituant organique ou minéral pouvant être éliminé à l'aide d'un agent alcalin.

**6.** Composé selon la revendication 5, dans lequel $X^2$ est le radical $\beta$-chloroéthyle, $\beta$-sulfatoéthyle, $\beta$-phosphatoéthyle ou $\beta$-thiosulfatoéthyle.

**7.** Composé selon la revendication 5 ou 6, dans lequel R est un hydrogène.

**8.** Composé selon la revendication 5 ou 6, dans lequel R' est un radical alkyle ayant de 1 à 4 atomes de carbone, éventuellement substitué par des radicaux phényle, ou encore est un radical phényle éventuellement substitué par un ou plusieurs substituants choisis parmi les halogènes et les radicaux nitro, alcoxy ayant de 1 à 4 atomes de carbone, phénoxy et alkyle ayant de 1 à 4 atomes de carbone.

**9.** Composé selon au moins l'une des revendications 5, 7 et 8, dans lequel $X^2$ est le radical $\beta$-sulfatoéthyle.

**10.** Procédé pour préparer un composé selon la revendication 5, caractérisé en ce qu'on fait réagir un composé acylamino-2 naphtalène de formule générale (2A)

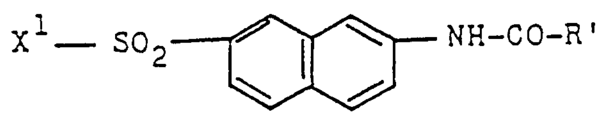

(2A)

dans laquelle R' est tel que défini dans la revendication 5 et $X^1$ a l'une des significations de $X^2$ ou encore est le groupe $\beta$-hydroxyéthyle, avec un agent de sulfonation pour former un composé de formule (5A) dans laquelle R est -CO-R',

puis on procède à une hydrolyse, si on souhaite former un composé de formule (5A) dans laquelle R est un hydrogène, du composé de formule (5A) dans laquelle R est -CO-R', en diluant à l'eau jusqu'à obtention d'une solution d'acide sulfurique à 85 à 100 %, puis chauffage à une température de 80 à 120 °C et, si on souhaite préparer un composé de formule (5A) dans laquelle $X^2$ est un groupe vinyle, en faisant réagir le composé obtenu de formule (5A) avec un agent alcalin.

**11.** Utilisation d'un composé de formule (5A), mentionné et défini dans l'une quelconque des revendications 5 à 8, en tant que composé de départ pour préparer un colorant réactif sur les fibres, en particulier quand R est un hydrogène, en tant que composant de diazotation pour préparer un colorant azoïque.

**12.** Colorant monoazoïque ou disazoïque de formule générale (6a) ou (6b)

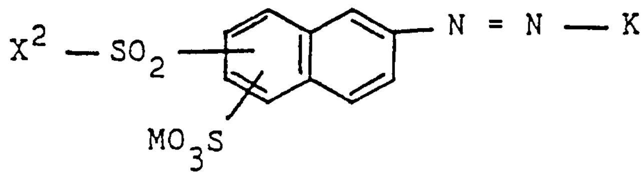

(6a)

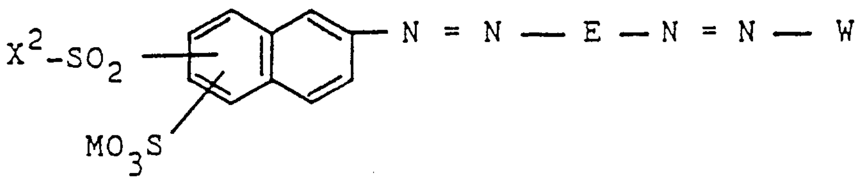

(6b)

où :

M est un hydrogène ou un métal alcalin,

$X^2$ est le groupe vinyle ou un groupe de formule $-CH_2-CH_2-Z$, où Z est un substituant organique ou minéral pouvant être éliminé au moyen d'un agent alcalin, et

le groupe sulfo $-SO_3M$ est en position 7 si le groupe $X^2-SO_2-$ est en position 5, ou bien le groupe sulfo est en position 5 si le groupe $X^2-SO_2-$est en position 7, ou bien

K est le résidu d'un composant de copulation, en particulier de la série du benzène, du naphtalène, de l'acide acétoacétique, de l'arylamine, de la pyrazolone et de la pyridone,

W est le radical K défini ci-dessus, ou encore est un radical D, D représentant un groupe de formule (8)

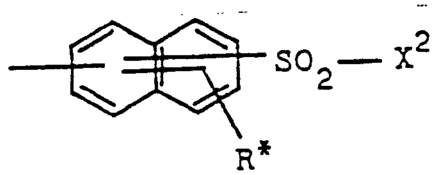

(8)

dans laquelle $X^2$ est tel que défini ci-dessus, et R* est un hydrogène ou le radical sulfo, ou encore D est le radical phényle ou naphtyle, chacun étant éventuellement substitué par 1, 2 ou 3 substituants choisis indépendamment les uns des autres parmi les radicaux chloro, fluoro, bromo, sulfo, carboxy, alcoxy ayant de 1 à 4 atomes de carbone, alkyle ayant de 1 à 4 atomes de carbone, benzoylamino, sulfobenzoylamino, alcanoylamino ayant de 2 à 5 atomes de carbone, N-alkylamino ayant de 1 à 4 atomes de carbone, N,N-dialkylamino ayant de 1 à 4 atomes de carbone dans chaque fragment alkyle et [chloro-5 phénylamino-3 s-triazinyl-1]-amino, le fragment benzène de ce dernier groupe étant éventuellement substitué par des radicaux sulfo, carboxy, chloro, méthyle, éthyle, méthoxy, éthoxy et/ou nitro et étant éventuellement substitué par un ou deux groupes $-SO_2-X^2$ tels que définis ci-dessus,

et

E représente un radical phénylène-1,4 ou naphtylène-1,4 éventuellement substitué par un ou deux substituants choisis indépendamment l'un de l'autre parmi les radicaux chloro, fluoro, bromo, sulfo, carboxy, alcoxy ayant de 1 à 4 atomes de carbone, alkyle ayant de 1 à 4 atomes de carbone, benzoylamino, sulfobenzoylamino, alcanoylamino ayant de 2 à 5 atomes de carbone, uréido et N,N-dialkylamino ayant de 1 à 4 atomes de carbone dans chaque fragment alkyle, ou encore est un radical naphtylène-2,6 ou -2,7 substitué en-position 1 par un groupe hydroxy et substitué par un ou deux groupes sulfo, ou bien

E représente un radical hydroxy-1 amino-8 disulfo-3,6 ou -4,6 naphtylène-2,7 ;

ou un de ses colorants à complexe métallifère.

**13.** Composé azoïque selon la revendication 12, dans lequel $X^2$ est l'un des radicaux $\beta$-chloroéthyle, $\beta$-sulfatoéthyle, $\beta$-phosphatoéthyle ou $\beta$-thiosulfatoéthyle.

**14.** Composé azoïque selon la revendication 12 ou 13, dans lequel $X^2$ est le radical $\beta$-sulfatoéthyle.

**15.** Composé monoazoïque selon au moins l'une des revendications 12 à 14, de formule générale (6a), dans laquelle K est un groupe de formule générale (7a)

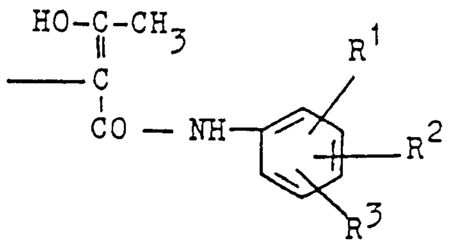

(7a)

dans laquelle $R^1$, $R^2$ et $R^3$ ont des significations qui peuvent être identiques les unes aux autres ou différentes les unes des autres, et $R^1$ est un hydrogène ou un radical hydroxy ou $-SO_2-X^2$ où $X^2$ a l'une des significations ci-dessus, $R^2$ est un hydrogène ou un radical chloro, bromo, alkyle ayant de 1 à 4 atomes de carbone, alcoxy ayant de 1 à 4 atomes de carbone ou sulfo, et $R^3$ est un hydrogène ou un radical chloro, alkyle ayant de 1 à 4 atomes de carbone, alcoxy ayant de 1 à 4 atomes de carbone ou sulfo,
ou un de ses complexes métallifères.

**16.** Composé monoazoïque selon au moins l'une des revendications 12 à 14, de formule générale (6a), dans laquelle K est un groupe ayant la formule générale (7b)

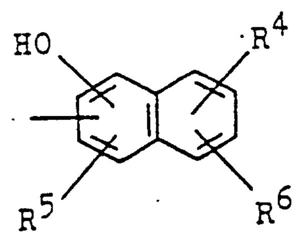

(7b)

dans laquelle la liaison libre se trouve en position ortho par rapport au groupe hydroxy, $R^4$ est un hydrogène ou un radical alkyle ayant de 1 à 4 atomes de carbone, alcoxy ayant de 1 à 4 atomes de carbone, sulfo, amino, hydroxy, benzoylamino, sulfobenzoylamino, alcanoylamino ayant de 2 à 5 atomes de carbone, N-alkylamino ayant de 1 à 4 atomes de carbone ou phénylamino-3 chloro-5 s-triazine ylamino-1, le fragment benzène de ce dernier groupe étant éventuellement substitué par des radicaux sulfo, carboxy, chloro, méthyle, éthyle, méthoxy, éthoxy et/ou nitro, de préférence par au moins un groupe nitro, $R^5$ est un hydrogène ou le radical sulfo, et $R^6$ est un hydrogène ou le radical sulfo, $R^4$, $R^5$ et $R^6$ pouvant avoir des significations identiques les unes aux autres ou différentes les unes des autres,
ou un de ses complexes métallifères.

**17.** Composé monoazoïque selon au moins l'une des revendications 12 à 14, de formule générale (6a), dans laquelle K est un groupe ayant la formule générale (7c)

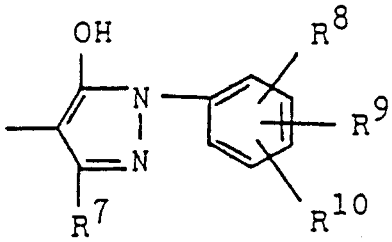

(7c)

dans laquelle $R^7$ est un radical alkyle ayant de 1 à 4 atomes de carbone, tel que le radical méthyle, un radical alcoxy ayant de 1 à 4 atomes de carbone, carboxy ou carbalcoxy ayant de 2 à 5 atomes de carbone, $R^8$ est un hydrogène ou un radical sulfo, alkyle ayant de 1 à 4 atomes de carbone, alcoxy ayant de 1 à 4 atomes de carbone, chloro, bromo, hydroxy ou $-SO_2-X^2$, $R^9$ est un hydrogène ou un radical sulfo, alkyle ayant de 1 à 4 atomes de carbone, alcoxy ayant de 1 à 4 atomes de carbone ou chloro, et $R^{10}$ est un hydrogène ou le radical sulfo,
ou un de ses complexes métallifères.

**18.** Composé monoazoïque selon au moins l'une des revendications 12 à 14, de formule générale (6a), dans laquelle K est un groupe ayant la formule générale (7d)

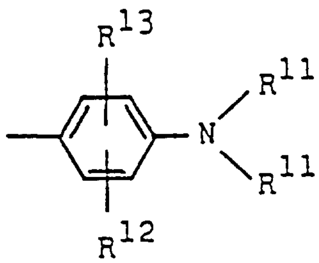

(7d)

dans laquelle chaque radical $R^{11}$ est, indépendamment de l'autre, choisi parmi l'hydrogène et les radicaux alkyle ayant de 1 à 4 atomes de carbone, éventuellement substitués par des radicaux sulfo, carboxy, hydroxy, sulfato ou alcoxy ayant de 1 à 4 atomes de carbone, $R^{12}$ est un radical hydroxy, chloro, bromo, alkyle ayant de 1 à 4 atomes de carbone, alcoxy ayant de 1 à 4 atomes de carbone, sulfo, alkylamino ayant de 1 à 4 atomes de carbone, alcanoylamino ayant de 2 à 5 atomes de carbone, uréido et $-SO_2-X^2$, et $R^{13}$ est un hydrogène ou un radical chloro, alkyle ayant de 1 à 4 atomes de carbone, alcoxy ayant de 1 à 4 atomes de carbone ou sulfo.

**19.** Colorant monoazoïque selon au moins l'une des revendications 12 à 14, dans lequel K est un groupe de formule (7e) ou (7f)

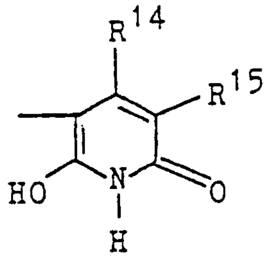

(7e)

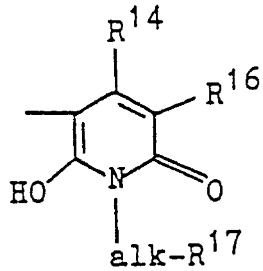

(7f)

dans laquelle

- $R^{14}$ est un hydrogène ou un radical alkyle ayant de 1 à 4 atomes de carbone, tel que les radicaux méthyle et phényle,
- $R^{15}$ est un hydrogène ou un radical chloro, bromo, sulfo, cyano, carbamoyle, carboxy ou

sulfamoyle, de préférence un hydrogène ou le radical carbamoyle,

$R^{16}$ est un hydrogène ou un radical chloro, bromo, sulfo, cyano, carbamoyle, carboxy ou sulfamoyle, de préférence un hydrogène ou le radical carbamoyle,

et

$R^{17}$ est un radical hydroxy, sulfo, sulfato, amino, alkylamino ayant de 1 à 4 atomes de carbone, alcoxy ayant de 1 à 4 atomes de carbone ou chloro, de préférence le radical méthoxy ou sulfo.

**20.** Colorant monoazoïque selon au moins l'une des revendications 12 à 14, dans lequel K est un groupe de formule générale (7g)

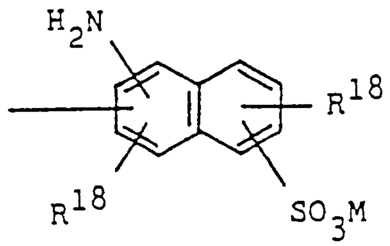

(7g)

dans laquelle la liaison libre se trouve en position ortho ou para par rapport au groupe amino, et chaque radical $R^{18}$ est, indépendamment de l'autre, choisi parmi l'hydrogène et le radical sulfo, et M est tel que défini ci-dessus.

**21.** Colorant monoazoïque à complexe de cuivre 1:1, de cobalt 1:2 ou de chrome 1:2 selon au moins l'une des revendications 12 à 14, du composé de formule générale (6A)

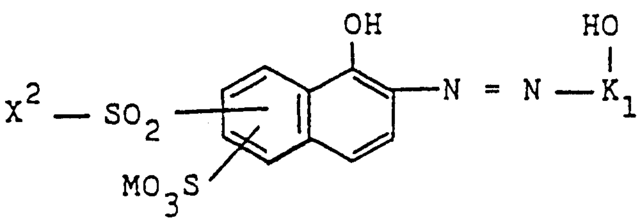

(6A)

dans laquelle M et $X^2$, ainsi que les positions des groupes $X^2$-$SO_2$- et $MO_3S$- sont tels que définis dans la revendication 12 pour la formule (6a), et $K_1$ est un fragment éventuellement substitué d'un agent de copulation hydroxylé choisi parmi la série mentionnée dans la revendication 12 pour le radical K, le groupe hydroxy de $K_1$ se trouvant en position ortho ou vicinale par rapport au groupe azoïque.

**22.** Colorant monoazoïque à complexe de cuivre 1:1 selon la revendication 21, dans lequel le radical -$K_1$-OH est un groupe représenté par l'une des formules générales (7a), (7b) et (7c) telles que définies dans les revendications 15 à 17.

**23.** Colorant monoazoïque à complexe de cuivre selon la revendication 21, ayant la formule générale (6B)

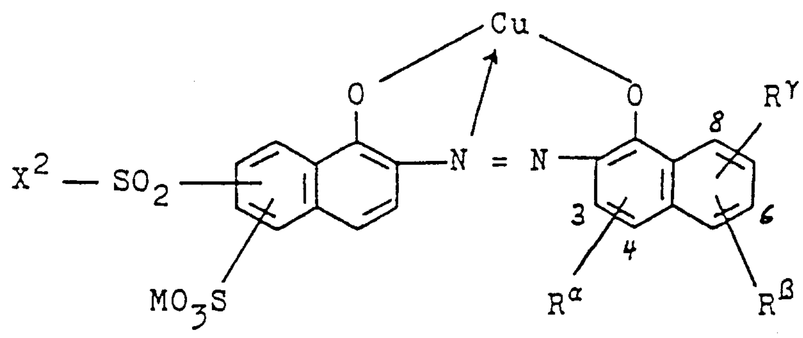

(6B)

dans laquelle $X^2$-$SO_2$- se trouve en position 5 ou 7 si le groupe $MO_3S$- est respectivement en position 7 ou 5, $R^\alpha$ est un radical sulfo en position 4, et $R^\beta$ et $R^\gamma$ sont chacun un hydrogène, ou encore $R^\alpha$ est un radical sulfo en position 3, $R^\beta$ est un radical sulfo en position 6 et $R^\gamma$ est un hydrogène, ou encore $R^\alpha$ est un radical sulfo en position 3, $R^\beta$ est un radical sulfo en position 6 et $R^\gamma$ est un radical acétylamino en position 8.

**24.** Procédé pour préparer un composé monoazoïque de formule (6a) selon la revendication 12 ou un composé disazoïque de formule (6b) selon la revendication 12, le radical W ayant cependant la signification de K, et le radical E représentant cependant le radical phénylène-1,4 ou naphtylène-1,4 éventuellement substitué, ou encore le radical hydroxy-1 mono- ou disulfonaphtylène2,6 ou -2,7 selon la revendication 12, caractérisé en ce qu'on diazote un aminonaphtalène de formule générale (5)

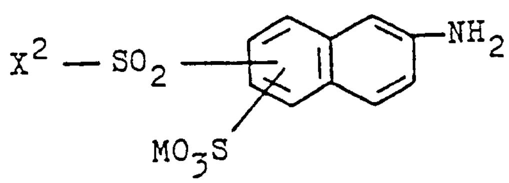

(5)

où M et $X^2$ sont tels que définis dans la revendication 12, et le substituant $X^2$-$SO_2$- se trouve en position 5 si le groupe $MO_3S$- est en position 7, ou encore le substituant $X^2$-$SO_2$- se trouve en position 7 si le groupe $MO_3S$-est en position 5,
ou encore un composé de formule générale (9)

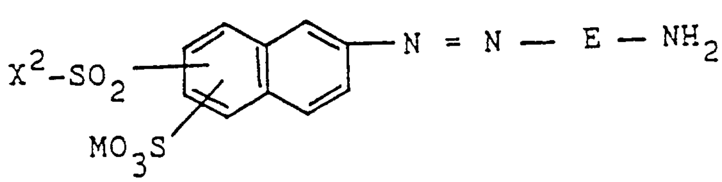

(9)

dans laquelle $X^2$, M et E sont tels que définis ci-dessus, et on procède à une copulation avec un composé de formule générale H-K dans laquelle K est tel que défini dans l'une quelconque des revendications 12 à 20.

**25.** Procédé pour préparer un composé disazoïque de formule générale (6b) selon la revendication 12, le radical W ayant cependant la signification de D et le radical E représentant cependant le groupe hydroxy-1 amino-8 disulfo-3, 6 naphtylène-2, 7 ou hydroxy-1 amino-8 disulfo4,6 naphtylène-2,7, caractérisé en ce qu'on copule un sel de diazonium d'un composé amino de formule (5)

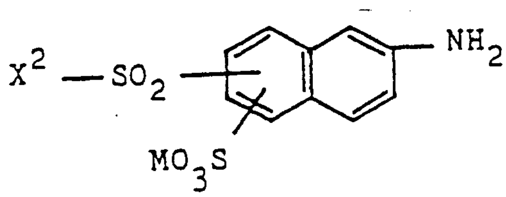

(5)

dans laquelle M et $X^2$ sont tels que définis dans la revendication 12, et le substituant $X^2$-$SO_2$- se trouve en position 5 si le groupe $MO_3S$- est en position 7, ou encore le substituant $X^2$-$SO_2$- se trouve en position 7 si le groupe $MO_3S$- est en position 5,
et un sel de diazonium d'un composé amino de formule générale $H_2N$-D dans laquelle D a l'une des significations données dans la revendication 12, et ce dans un ordre quelconque voulu, avec un composé de copulation de formule générale H-E-H où E est le radical hydroxy-1 amino-8 disulfo-3,6 ou -4,6 naphtylène-2,7, la réaction de copulation étant d'abord mise en oeuvre à un pH fortement acide puis sur un intervalle de pH faiblement acide à très faiblement alcalin.

**26.** Procédé pour préparer un complexe métallifère, de préférence un complexe de cuivre 1:1, d'un composé monoazoïque ou disazoïque selon au moins l'une des revendications 12 à 23, caractérisé en ce que le colorant monoazoïque ou disazoïque non métallifère selon l'une des revendications 12 à 19 est mis à réagir avec un agent donneur de métaux, de préférence un agent donneur de cuivre, en présence d'un oxydant.

**27.** Utilisation d'un composé azoïque selon au moins l'une des revendications 12 à 23 en tant que colorant pour teindre ou imprimer des matériaux fibreux contenant des groupes hydroxy et/ou carboxamido.

**28.** Procédé pour teindre ou imprimer un matériau fibreux contenant des groupes hydroxy et/ou carboxamido par application d'un colorant sur le matériau et fixage au moyen de chaleur et/ou d'un agent fixant les acides, caractérisé en ce que le colorant utilisé est un composé azoïque selon au moins l'une des revendications 12 à 23.